(19) **Europäisches Patentamt European Patent Office Office européen des brevets**

(11) **EP 4 200 004 B1**

(12) **EUROPEAN PATENT SPECIFICATION**

(45) Date of publication and mention
of the grant of the patent:
**17.06.2026 Bulletin 2026/25**

(21) Application number: **21860659.8**

(22) Date of filing: **13.08.2021**

(51) International Patent Classification (IPC):
**A61M 16/10** (2006.01)    **A61M 16/16** (2006.01)
**A61M 16/08** (2006.01)

(52) Cooperative Patent Classification (CPC):
**A61M 16/024; A61M 13/003; A61M 16/109;**
**A61M 16/1095; A61M 16/16;** A61M 16/0051;
A61M 16/0069; A61M 16/0875; A61M 16/1085;
A61M 16/161; A61M 16/162; A61M 2016/0027;
A61M 2016/0039; A61M 2205/17; A61M 2205/18;
(Cont.)

(86) International application number:
**PCT/IB2021/057457**

(87) International publication number:
**WO 2022/043814 (03.03.2022 Gazette 2022/09)**

(54) **OVER-ENTHALPY PROTECTION IN HUMIDIFIER SYSTEMS**

ÜBERENTHALPIESCHUTZ IN BEFEUCHTERSYSTEMEN

PROTECTION CONTRE LA SURENTHALPIE DANS DES SYSTÈMES D'HUMIDIFICATEUR

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **24.08.2020  US 202063069483 P**

(43) Date of publication of application:
**28.06.2023  Bulletin 2023/26**

(60) Divisional application:
**26177468.1**

(73) Proprietor: **Fisher & Paykel Healthcare Limited**
**East Tamaki**
**Auckland 2013 (NZ)**

(72) Inventors:
• **YU, Yintao**
**Auckland, 2013 (NZ)**

• **LIANG, Wenjie Robin**
**Auckland, 2013 (NZ)**

(74) Representative: **Treeby, Philip David William et al**
**Maucher Jenkins**
**Seventh Floor Offices**
**Artillery House**
**11-19 Artillery Row**
**London SW1P 1RT (GB)**

(56) References cited:
EP-A2- 1 634 614        WO-A1-01/13981
WO-A1-2017/036500       WO-A1-2017/043981
WO-A1-2020/043672       US-A1- 2006 113 690
US-A1- 2010 132 707     US-A1- 2011 023 874
US-A1- 2020 009 347     US-A1- 2020 009 347
US-A1- 2020 016 361     US-A1- 2020 114 098

(52) Cooperative Patent Classification (CPC): (Cont.)
A61M 2205/3334; A61M 2205/3368;
A61M 2205/3372; A61M 2205/3386;
A61M 2205/3592; A61M 2205/3653;
A61M 2205/505; A61M 2205/581; A61M 2205/583;
A61M 2205/75

**Description**

FIELD OF THE DISCLOSURE

The present disclosure relates to respiratory and/or surgical humidifier systems configured to supply humidified gases to a user (for example, a patient).

BACKGROUND

Respiratory apparatuses and surgical insufflators provide a flow of pressurized gases through a conduit system to a patient. For a range of applications using these and similar devices, it is beneficial to humidify gases the supplied gases. These applications include where the gases are for breathing by the user and/or where the gases are being supplied during surgery to a surgical site in a patient.

In the case of breathing gases in a noninvasive mode when the inspired gases pass through the upper airway, such as when gases are delivered to the user via a face or nasal mask, the humidity increases user comfort, improves the user's tolerance to the noninvasive ventilation (NIV), and the humidified gases are less prone to drying out the tissues (for example, the nasal mucosa) of the airway of the user. In the case of an invasive mode when the gases delivered to the user bypass the upper airway, humidification of the gases has been found to improve user comfort and provide physiological benefits, such as improved mucus transport, can be necessary for user safety, such as for preventing airway obstruction due to inspissation of airway secretion, disruption of the airway epithelium, and/or for improving post-operative outcomes. In the case of surgical gases being delivered to a surgical site (for example, an open surgery site) in the patient, humidification of the gases can also improve post-operative outcomes. Humidification of surgical gases can also prevent or mitigate desiccation of tissue, such as mesothelium.

In the case of high flow therapy, humidified gases are delivered to the user at high flows through an unsealed patient interface. The user may be spontaneously breathing during high flow therapy. In some situations, gases can also be delivered at a high flow rate to a user who may be apneic, such as under anesthesia. A high flow system or high flow therapy apparatus with a humidifier can be used to deliver high flow gases and the therapy apparatus can control characteristics, such as for example characteristics of the gases flow, including the flow rate, temperature, pressure, humidity, supplementary gases concentration, and the like. The use of high flow rate gases delivery can help to push the gases flow and hence oxygen deeper into the patient's airways. A high flow rate of gases also helps flushing of the airways, and flushing of carbon dioxide, which also can help to push oxygen/respiratory gases deeper into the airways.

In the case of positive airway pressure therapy (PAP) therapy, a PAP therapy apparatus that includes a blower and a humidifier can be used to provide pressure therapy, for example, continuous positive airway pressure therapy (CPAP), to the user.

US 2020/009347 A1 relates to a humidification apparatus that can include a controller, a heater plate, and a breathing tube with a tube heater. Based on heater plate target temperature and tube heater target power, the controller generates and passes control voltages to the heater plate and breathing tube heater respectively to control them to attain and maintain the target heater plate temperature and breathing tube power to achieve the target temperature/humidity at the end of a hose. The controller can provide heater plate temperature, among other things, to a neural network model or equations that output a target power for the breathing tube.

SUMMARY

The invention is defined in the appended independent claim 1. Preferred embodiments are matter of the dependent claims.

A respiratory humidifier system can support an invasive mode and/or be capable of delivering a higher humidity level to a user and/or receive a higher flow rate of gases from a gases source (for example, the ventilator). A surgical humidifier system can provide humidified surgical gases to a surgical site, for example, a peritoneal cavity, in a patient. An increased heater plate power can be required in the respiratory and/or surgical humidifier systems to support the higher humidity mode. In addition to being designed to keep the condition of the gases delivered to the user at a desired humidity and/or temperature to prevent condensation from forming within an inspiratory conduit, the systems disclosed herein can also protect the user by reducing a risk of an undesirable, dangerous, and/or harmful level of enthalpy, that is, an over-enthalpy scenario, from being delivered to the user. Enthalpy can be defined as a thermodynamic quantity equivalent to the total heat content of a system. Nonlimiting factors that affect enthalpy of the delivered gases include temperature and/or humidity. Enthalpy at or near the user should be kept below a predetermined level to prevent discomfort or in some cases serious harm to the user. The enthalpy level can be of a greater concern at low flow rates (for example, below about 7 L/min, or between about 4 L/min and about 7 L/min, or otherwise) as the heat energy is more concentrated per unit of flow. When the flow rate is higher, enthalpy can be less of or not a concern as the gases at a higher flow rate can sufficiently remove the built-up energy from the respiratory and/or surgical systems.

Enthalpy may become a problem when there is a significant change in the operational state of the respiratory humidifier system. For example, when the respiratory humidifier system is operating with a relatively high flow and transitions quickly to a relatively low flow, there can be a significant increase in enthalpy. Measuring, predicting and controlling enthalpy prevents a potentially dangerous increase in enthalpy. This can be done, for example, but using a wide range of sensors throughout the system to measure actual conditions, in addition to a controller programmed to prevent over-enthalpy scenarios. Measuring, predicting and controlling enthalpy becomes much more difficult when the respiratory humidifier system has only a limited number of sensors. For example, if the respiratory humidifier system does not have any sensors downstream from the humidification chamber, it is difficult to know how much enthalpy is actually reaching the patient. Further, if the respiratory system only has sensors in the flow generator (flow, motor speed or pressure) and/or in the humidifier (for example heater plate temperature sensors), the determination of an over-enthalpy system may also be difficult. Accordingly, the present Application provides configurations which protect a patient from an over-enthalpy scenario. These configurations can be for systems with a wide ranch of sensors throughout the entire respiratory humidification system, as well as specific configurations for respiratory humidification systems with few or very limited sensors as will be discussed below in more detail.

An example respiratory or surgical humidifier system configured to deliver a flow of gases to a user can comprise a base unit comprising: a heater plate including one or more heater plate heating elements; and a hardware controller configured to cause energization of the one or more heating elements of the heater plate, the base unit configured to receive a humidifier chamber including a thermally conductive base such that the thermally conductive base contacts the heater plate, the humidifier chamber configured to hold a volume of water, wherein the hardware controller is configured to be in electronic communication with a conduit heating element in a conduit (for example a inspiratory or insufflation conduit) configured to transport the gases from the humidifier chamber to a patient interface, the system configured to determine and/or apply a power limit on the conduit heating element based at least in part on one or more parameters of the heater plate.

Alternatively, in a configuration, the system can be configured to determine and/or apply a power limit on the conduit heating element based at least in part on a water temperature of the water held in the humidifier chamber.

In a configuration, the system can further comprise a heater plate temperature sensor configured to measure a heater plate temperature and the system can be configured to determine and/or apply the power limit based at least in part on the heater plate temperature.

In a configuration, the system can further comprise an ambient temperature sensor configured to measure an ambient temperature and the system can be configured to determine and/or apply a power limit on the conduit heating element based at least in part on the ambient temperature.

In a configuration, the heater plate temperature sensor and/or the ambient temperature sensor can comprise one or more thermistors.

In a configuration, the one or more thermistors can comprise at least one negative temperature coefficient (NTC) thermistor.

In a configuration, the system can be configured to determine and/or apply the power limit based at least in part on a heater plate power.

In a configuration, the power limit on the conduit heating element can be non-binary.

**In** a configuration, the power limit can be configured to keep a user end temperature of the gases under a first temperature limit.

In a configuration, the first temperature limit can be from 32°C to 45°C.

In a configuration, the first temperature limit can be 40°C.

In a configuration, the first temperature limit can be a first dew point temperature limit.

In a configuration, the power limit can be configured to keep a user end enthalpy of the gases under a first enthalpy limit on a specific enthalpy for dry air.

In a configuration, the first enthalpy limit can be from 122 kJ/m$^3$ to 216 kJ/m$^3$.

In a configuration, the first enthalpy limit can be from 195 kJ/m$^3$ to 205 kJ/m$^3$.

In a configuration, the system can be configured to determine and/or apply the power limit independent of control of energization of the one or more heating elements of the heater plate.

In a configuration, the system can be configured to determine and/or apply the power limit using the hardware controller.

In a configuration, the hardware controller can comprise a central processing unit (CPU).

In a configuration, the system can be configured to determine and/or apply the power limit based at least in part on a value indicative of a parameter indicative of a flow rate of the flow of gases.

In a configuration, the power limit can be applied in response to the heater plate temperature exceeds a heater plate temperature threshold.

In a configuration, the system can be further configured to output a power to the conduit heating element, the power substantially not exceeding the power limit.

In a configuration, the power limit can be a limit on a duty cycle of the conduit heating element.

In a configuration, the system can be configured to determine and/or apply the power limit without requiring an electrical current input of the conduit heating element.

In a configuration, the system can be configured to determine and/or apply the power limit without requiring input from sensors in a gases flow pathway.

In a configuration, the system can be configured to determine and/or apply the power limit without requiring input from sensors located at or near a chamber outlet and/or a patient end.

In a configuration, the system may not comprise a sensor at or near the chamber outlet and/or the patient end.

In a configuration, applying the power limit does not comprise turning off the power to the conduit heating element and/or the one or more heater plate heating elements.

In a configuration, applying the power limit can further comprise turning off the power to the conduit heating element.

In a configuration, the power limit can be further configured to keep the user end enthalpy under a second enthalpy limit on the specific enthalpy for dry air, wherein the second enthalpy limit can be higher than the first enthalpy limit.

In a configuration, the second enthalpy limit can be from 197 kJ/m$^3$ to 441 kJ/m$^3$.

In a configuration, the second enthalpy limit can be from 195 kJ/m$^3$ to 205 kJ/m$^3$.

In a configuration, the second enthalpy limit can be for redundancy over the first enthalpy limit.

In a configuration, the second enthalpy limit can be configured to protect the user from more significant harm than the first enthalpy limit.

In a configuration, the power limit can be further configured to keep the user end temperature under a second temperature limit, wherein the second temperature limit is higher than the first temperature limit.

In a configuration, the second temperature limit can be from 43°C to 63°C.

In a configuration, the second temperature limit can be from 43°C to 45°C.

In a configuration, the second temperature limit can be a second dew point temperature limit.

In a configuration, the second temperature limit can be for redundancy over the first temperature limit.

In a configuration, the second temperature limit can be configured to protect the user from more significant enthalpy level than the first temperature limit.

In a configuration, the system can be configured to apply the power limit to keep the user end temperature under the second temperature limit or the second enthalpy limit using a CPU or electronic circuitry outside of the CPU.

In a configuration, applying the power limit to keep the user end temperature or enthalpy under the second temperature limit or the second enthalpy limit can comprise turning off the power to the conduit heating element.

In a configuration, the power limit to keep the user end temperature or enthalpy under the second temperature limit or the second enthalpy limit can be applied based at least in part on the heater plate temperature.

In a configuration, the power limit to keep the user end temperature or enthalpy under the second temperature limit or the second enthalpy limit can be applied based at least in part on the ambient temperature.

In a configuration, the power limit to keep the user end temperature or enthalpy under the second temperature limit or the second enthalpy limit can decrease as the ambient temperature increases.

In a configuration, the power limit configured to keep the user end temperature or enthalpy under the second temperature limit or the second enthalpy limit can be applied only upon the heater plate temperature exceeding a second heater plate temperature threshold.

In a configuration, the power limit configured to keep the user end temperature or enthalpy under the second temperature limit or the second enthalpy limit can be determined and/or applied based at least in part on the heater plate temperature when the humidifier system is in steady state.

In a configuration, a heater plate power can be assumed to have a fixed correlation to a difference between the heater plate temperature and a temperature of the volume of water held in the humidifier chamber.

In a configuration, the power limit to keep the user end temperature or enthalpy under the second temperature limit or the second enthalpy limit can be applied by triggering a first protection circuit that comprises a comparator.

In a configuration, the power limit to keep the user end temperature or enthalpy under the second temperature limit or the second enthalpy limit can be based in part on a heater plate power.

In a configuration, the first protection circuit can apply the power limit based in part on the heater plate power.

In a configuration, the system can further comprise a second protection circuit comprising a second comparator, the second protection circuit applying the power limit based in part on the heater plate power.

In a configuration, a temperature of the volume of water held in the humidifier chamber when the humidifier system is in transient state can be estimated based at least in part on the heater plate power.

In a configuration, the temperature of the volume of water when the humidifier system is in the transient state can be estimated further based in part on the heater plate temperature.

In a configuration, the power limit to keep the user end temperature or enthalpy under the second temperature limit or the second enthalpy limit can decrease when the heater plate power decreases.

In a configuration, the system can further comprise the inspiratory conduit.

In a configuration, the system can further comprise an expiratory conduit.

In a configuration, the power limit on the conduit heating element can be triggered when the flow of gases is at below 7 L/min.

In a configuration, the power limit on the conduit heating element can be triggered when the flow of gases is between 4 L/min and 7 L/min.

An example respiratory or surgical humidifier system configured to deliver a flow of gases to a user can comprise a base unit comprising: a heater plate including one or more heater plate heating elements; and a hardware controller configured to cause energization of the one or more heating elements of the heater plate; the base unit configured to receive a humidifier chamber including a thermally conductive base such that the thermally conductive base contacts the heater plate, the humidifier chamber configured to hold a volume of water, wherein the hardware controller can be in electronic communication with a conduit heating element in an inspiratory conduit configured to transport gases from the humidifier chamber to a patient interface, the hardware controller programmed to determine and/or apply a power limit on the conduit heating element based at least in part on one or more parameters of the heater plate that includes a parameter indicative of a flow rate of the flow of gases.

In a configuration, the system can further comprise a heater plate temperature sensor configured to measure a heater plate temperature and the hardware controller can be programmed to determine and/or apply the power limit based at least in part on the heater plate temperature.

In a configuration, the system can further comprise an ambient temperature sensor configured to measure an ambient temperature and the hardware controller programmed to determine and/or apply a power limit on the conduit heating element based at least in part on the ambient temperature.

In a configuration, the power limit can be configured to keep a user end temperature of the gases under a temperature limit.

In a configuration, the temperature limit can be from 32°C to 45°C.

In a configuration, the temperature limit can be from 42°C to 45°C.

In a configuration, the temperature limit can be a dew point temperature limit.

In a configuration, the power limit can be configured to keep a user end enthalpy of the gases under a first enthalpy limit on a specific enthalpy for dry air.

In a configuration, the first enthalpy limit can be from 122 kJ/m$^3$ to 216 kJ/m$^3$.

In a configuration, the first enthalpy limit can be from 195 kJ/m$^3$ to 205 kJ/m$^3$.

In a configuration, the hardware controller can be programmed to determine and/or apply the power limit independent of control of energization of the one or more heating elements of the heater plate.

In a configuration, the hardware controller can be programmed to determine and/or apply the power limit based at least in part on a heater plate power.

In a configuration, the hardware controller can be further programmed to apply the power limit in response to the heater plate temperature exceeding a heater plate temperature threshold.

In a configuration, the hardware controller can be further programmed to output a power to the conduit heating element, the power substantially not exceeding the power limit.

In a configuration, the power limit can be a limit on a duty cycle of the conduit heating element.

In a configuration, the hardware controller can be programmed to determine and/or apply the power limit without requiring a current input of the conduit heating element.

In a configuration, the system can be configured to determine and/or apply the power limit without requiring input from sensors in a gases flow pathway.

In a configuration, the system can be configured to determine and/or apply the power limit without requiring input from sensors located at or near a chamber outlet and/or a patient end.

In a configuration, the system may not comprise a sensor at or near the chamber outlet and/or the patient end.

In a configuration, applying the power limit does not comprise turning off the power to the conduit heating element.

In a configuration, applying the power limit can further comprise turning off the power to the conduit heating element and/or to the one or more heater plate heating elements.

In a configuration, the parameter indicative of the flow rate of the flow of gases can comprise a thermal conductivity value or a value indicative of a thermal conductivity between the heater plate and the flow of gases.

In a configuration, the power limit can comprise a power offset.

In a configuration, the power offset can be based at least in part on the parameter indicative of a flow rate of the flow of gases.

In a configuration, the power offset can be based at least in part on a difference between a threshold of the parameter indicative of a flow rate of the flow of gases and the parameter indicative of a flow rate of the flow of gases.

In a configuration, the power offset can be based at least in part on the heater plate temperature.

In a configuration, the power offset can be based at least in part on a difference between a heater plate temperature and a heater plate temperature threshold.

In a configuration, the power offset can be zero when the parameter indicative of a flow rate of the flow of gases is equal to or above the threshold of said parameter and the heater plate temperature is equal to or below the heater plate temperature threshold.

In a configuration, the power offset can be zero when a difference between the threshold of the parameter indicative of a flow rate of the flow of gases and the parameter indicative of a flow rate of the flow of gases is non-positive and/or the difference between the heater plate temperature and the heater plate temperature threshold is non-positive.

In a configuration, the power offset can be positive when the parameter indicative of a flow rate of the flow of gases is below the threshold of said parameter and the heater plate temperature is greater than the heater plate temperature threshold.

In a configuration, the power offset can be positive when the difference between the threshold of the parameter indicative of a flow rate of the flow of gases and the parameter indicative of a flow rate of the flow of gases is positive and the difference between the heater plate temperature and the heater plate temperature threshold is positive.

In a configuration, the system can further comprise the inspiratory conduit.

In a configuration, the system can further comprise an expiratory conduit.

In a configuration, the power limit on the conduit heating element can be triggered when the flow of gases is at below 7 L/min.

In a configuration, the power limit on the conduit heating element can be triggered when the flow of gases is between 4 L/min and 7 L/min.

An example respiratory or surgical humidifier system configured to deliver a flow of gases to a user can comprise a base unit comprising: a heater plate including one or more heater plate heating elements; a heater plate temperature sensor configured to measure a heater plate temperature; an ambient temperature sensor configured to measure an ambient temperature; and a hardware controller configured to cause energization of the one or more heating elements of the heater plate; and the base unit configured to receive a humidifier chamber including a thermally conductive base such that the thermally conductive base contacts the heater plate, the humidifier chamber configured to hold a volume of water, wherein the hardware controller can be programmed to determine a reduction in a flow rate of the gases based at least in part on one or more parameters of the heater plate.

In a configuration, the hardware controller can be programmed to determine the reduction in the flow rate without calculating a flow rate value.

In a configuration, the system does not comprise a flow sensor.

In a configuration, the hardware controller can be programmed to determine the reduction in the flow rate of the gases based at least in part on a heater plate power.

In a configuration, the hardware controller can be programmed to determine a reduction in the flow rate of the gases based at least in part on the heater plate temperature and/or the ambient temperature.

In a configuration, the hardware controller can be programmed to limit a power to a conduit heating element of an inspiratory conduit when the flow of gases is in a low flow state and the heater plate temperature exceeds a threshold, the inspiratory conduit configured to transport the gases from the humidifier chamber to a patient interface.

In a configuration, the system can further comprise an inspiratory conduit.

In a configuration, the system can further comprise an expiratory conduit.

In a configuration, the power limit on the conduit heating element can be triggered when the flow of gases is at below 7 L/min.

In a configuration, the power limit on the conduit heating element can be triggered when the flow of gases is between 4 L/min and 7 L/min.

In an example respiratory or surgical humidifier system configured to deliver a flow of gases to a user can comprise a base unit comprising: a heater plate including one or more heater plate heating elements; and a hardware controller configured to cause energization of the one or more heating elements of the heater plate, the base unit configured to receive a humidifier chamber including a thermally conductive base such that the thermally conductive base contacts the heater plate, the humidifier chamber configured to hold a volume of water, wherein the hardware controller can be configured to be in electronic communication with a conduit heating element in an inspiratory conduit configured to transport the gases from the humidifier chamber to a patient interface, the system configured to determine and/or apply a power limit on the conduit heating element based at least in part on a heater plate temperature and/or a heater plate power.

In a configuration, the system can further comprise a heater plate temperature sensor configured to measure a heater plate temperature and the system can be configured to determine and/or apply the power limit based at least in part on the heater plate temperature.

In a configuration, the system can further comprise an ambient temperature sensor configured to measure an ambient temperature and the system can be configured to determine and/or apply a power limit on the conduit heating element based at least in part on the ambient temperature.

In a configuration, the system can further comprise one or more thermistors.

In a configuration, the one or more thermistors can comprise at least one negative temperature coefficient (NTC) thermistor.

In a configuration, the system can be configured to apply the power limit using electronic circuitry outside the hardware controller.

In a configuration, the hardware controller can comprise a CPU.

In a configuration, a power limit can comprise a limit on a duty cycle.

In a configuration, the power limit can be configured to keep a user end temperature of the gases under a temperature limit.

In a configuration, the temperature limit can be from 43°C to 63°C.

In a configuration, the temperature limit can be from 43°C to 45°C.

In a configuration, the temperature limit can be a dew point temperature limit.

In a configuration, the power limit can be configured to keep the user end enthalpy under an enthalpy limit on a specific enthalpy for dry air.

In a configuration, the enthalpy limit can be from 197 kJ/m$^3$ to 441 kJ/m$^3$.

In a configuration, the enthalpy limit can be from 195 kJ/m$^3$ to 205 kJ/m$^3$.

In a configuration, the power limit can be for redundancy over a heater plate software and/or hardware safety feature.

In a configuration, the power limit can be configured to protect the user from more significant enthalpy level than the heater plate software and/or hardware safety feature.

In a configuration, the system can be configured to determine and/or apply a power limit on the conduit heating element based at least in part on a parameter indicative of a flow rate of the flow of gases.

In a configuration, the power limit can decrease as the ambient temperature increases.

In a configuration, the power limit can be applied in response to the heater plate temperature exceeding a temperature threshold.

In a configuration, the power limit can be applied based at least in part on the heater plate temperature when the humidifier system is in steady state.

In a configuration, a heater plate power can be assumed to have a fixed correlation to a difference between the heater plate temperature and a temperature of the volume of water held in the humidifier chamber.

In a configuration, the power limit can be applied by triggering a first protection circuit that comprises a comparator.

In a configuration, the first protection circuit can trigger the power limit based at least in part on a heater plate power.

In a configuration, the first protection circuit can trigger the power limit based at least in part on the heater plate power.

In a configuration, the system can further comprise a second protection circuit comprising a second comparator, the second protection circuit configured to apply the power limit based at least in part on the heater plate power.

In a configuration, the system can be configured to determine and/or apply the power limit without requiring input from sensors in a gases flow pathway.

In a configuration, the system can be configured to determine and/or apply the power limit without requiring input from sensors located at or near a chamber outlet and/or a patient end.

In a configuration, the system may not comprise a sensor at or near the chamber outlet and/or the patient end.

In a configuration, a temperature of the volume of water held in the humidifier chamber when the humidifier system is in transient state can be estimated based at least in part on the heater plate power.

In a configuration, the temperature of the volume of water when the humidifier system is in the transient state can be estimated further based in part on the heater plate temperature.

In a configuration, the power limit can decrease when the heater plate power decreases.

In a configuration, the system can further comprise the inspiratory conduit.

In a configuration, the system can further comprise an expiratory conduit.

In a configuration, the power limit on the conduit heating element can be triggered when the flow of gases is at below 7 L/min.

In a configuration, the power limit on the conduit heating element can be triggered when the flow of gases is between 4 L/min and 7 L/min.

An example respiratory or surgical humidifier system configured to deliver a flow of gases to a user can comprise a base unit comprising: a heater plate including one or more heater plate heating elements; a hardware controller configured to cause energization of the one or more heating elements of the heater plate, the base unit configured to receive a humidifier chamber including a thermally conductive base such that the thermally conductive base contacts the heater plate, the humidifier chamber configured to hold a volume of water, wherein the hardware controller can be configured to be in electronic communication with a conduit heating element in an inspiratory conduit configured to transport the gases from the humidifier chamber to a patient interface; and an over-enthalpy protection circuit configured to apply a power limit on a conduit heating element power based at least in part on a heater plate temperature and/or a heater plate power, the over-enthalpy protection circuit comprising a comparator.

In a configuration, the system can further comprise a heater plate temperature sensor configured to measure a heater plate temperature and the over-enthalpy protection circuit can be configured to apply the power limit based at least in part on the heater plate temperature.

In a configuration, the system can further comprise an ambient temperature sensor configured to measure an ambient temperature and the system can be configured to apply a power limit on the conduit heating element based at least in part on the ambient temperature.

In a configuration, the system can further comprise one or more thermistors.

In a configuration, the one or more thermistors can comprise at least one negative temperature coefficient (NTC) thermistor.

In a configuration, the power limit can comprise limiting limit on a duty cycle of the conduit heating element.

In a configuration, the over-enthalpy protection circuit can be configured to keep a user end temperature of the gases under a temperature limit.

In a configuration, the temperature limit can be from 43°C to 63°C.

In a configuration, the temperature limit can be from 43°C to 45°C.

In a configuration, the temperature limit can be a dew point temperature limit.

In a configuration, the temperature limit can be a dew point temperature limit.

In a configuration, the power limit can be configured to keep the user end enthalpy under an enthalpy limit.

In a configuration, the enthalpy limit can be from 195 kJ/m$^3$ to 441 kJ/m$^3$.

In a configuration, the enthalpy limit can be from 195 kJ/m$^3$ to 205 kJ/m$^3$.

In a configuration, the over-enthalpy protection circuit can be for redundancy over a heater plate software and/or hardware safety feature.

In a configuration, the power limit can be configured to protect the user from more significant enthalpy level than the software conduit heating element safety.

In a configuration, the power limit can decrease as the ambient temperature increases.

In a configuration, the over-enthalpy protection circuit can be configured to apply the power limit only upon the heater plate temperature exceeding a temperature threshold.

In a configuration, the over-enthalpy protection circuit can be configured to apply the power limit based at least in part on the heater plate temperature when the humidifier system is in steady state.

In a configuration, a heater plate power can be assumed to have a fixed correlation to a difference between the heater plate temperature and a temperature of the volume of water held in the humidifier chamber.

In a configuration, the system can further comprise a second over-enthalpy protection circuit including a second comparator.

In a configuration, the second over-enthalpy protection circuit can be configured to apply the power limit based at least in part on the heater plate power.

In a configuration, the second over-enthalpy protection circuit can be configured to apply the power limit based at least in part on the heater plate temperature.

In a configuration, the second over-enthalpy protection circuit can be configured to apply the power limit based at least in part on the ambient temperature.

In a configuration, the system can be configured to determine and/or apply the power limit without requiring input from sensors in a gases flow pathway.

In a configuration, the system can be configured to determine and/or apply the power limit without requiring input from sensors located at or near a chamber outlet and/or a patient end.

In a configuration, the system may not comprise a sensor at or near the chamber outlet and/or the patient end.

In a configuration, a temperature of the volume of water held in the humidifier chamber when the humidifier system is in transient state can be estimated based at least in part on the heater plate power.

In a configuration, the temperature of the volume of water when the humidifier system is in the transient state can be estimated further based in part on the heater plate temperature.

In a configuration, the power limit can decrease when the heater plate power decreases.

In a configuration, the system can further comprise the inspiratory conduit.

In a configuration, the system can further comprise an expiratory conduit.

In a configuration, the power limit on the conduit heating element can be triggered when the flow of gases is at below 7 L/min.

In a configuration, the power limit on the conduit heating element can be triggered when the flow of gases is between 4 L/min and 7 L/min.

An example respiratory or surgical humidifier system configured to deliver a flow of gases to a user, the system comprising: a base unit comprising: a heater plate including one or more heater plate heating elements; and a hardware controller configured to energize the one or more heating elements of the heater plate, the base unit configured to receive a humidifier chamber including a thermally conductive base such that the thermally conductive base contacts the heater plate, the humidifier chamber configured to hold a volume of water, wherein the hardware controller is configured to be in electronic communication with a conduit heating element in an inspiratory conduit configured to transport the gases from the humidifier chamber to a patient interface, the system configured to determine and/or apply a power limit on the conduit heating element based at least in part on one or more parameters of the heater plate.

In a configuration, determining and/or applying a power limit on the conduit heating element can comprise controlling power in the conduit heating element to, or below, the power limit.

In a configuration, determining and/or applying a power limit on the conduit heating element can comprise disabling a component of the system or the entire system if the power limit on the conduit heating element is exceeded.

In a configuration, disabling a component of the system can comprise disabling the conduit heating element.

In a configuration, disabling a component of the system can comprise disabling the heater plate.

In a configuration, the system can further comprise a heater plate temperature sensor configured to measure a heater plate temperature and the one or more parameters of the heater plate, which the system is configured to determine and/or apply the power limit based at least in part on, includes at least the heater plate temperature.

In a configuration, the system can further comprise an ambient temperature sensor configured to measure an ambient temperature and the system is configured to determine and/or apply a power limit on the conduit heating element based at least in part on the ambient temperature.

In a configuration, the heater plate temperature sensor and/or the ambient temperature sensor can comprise one or more thermistors.

In a configuration, the one or more thermistors can comprise at least one negative temperature coefficient (NTC) thermistor.

In a configuration, the one or more parameters of the heater plate, which the system is configured to determine and/or apply the power limit based at least in part on, can include at least a heater plate power.

In a configuration, heater plate power can be controlled by heater plate voltage.

In a configuration, the power limit on the conduit heating element can be non-binary.

In a configuration, the power limit can be configured to keep a user end temperature of the gases under a first temperature limit.

In a configuration, the first temperature limit can be from 32°C to 45°C.

In a configuration, the first temperature limit can be 40°C.

In a configuration, the first temperature limit can be a first dew point temperature limit.

In a configuration, the power limit can be configured to keep a user end enthalpy of the gases under a first enthalpy limit on a specific enthalpy for dry air.

In a configuration, the first enthalpy limit can be from 122 kJ/m$^3$ to 216 kJ/m$^3$.

In a configuration, the first enthalpy limit can be from 195 kJ/m$^3$ to 205 kJ/m$^3$.

In a configuration, the system can be configured to determine and/or apply the power limit independent of control of energization of the one or more heating elements of the heater plate.

In a configuration, the system can be configured to determine and/or apply the power limit using the hardware controller.

In a configuration, the hardware controller can comprise a central processing unit (CPU).

In a configuration, the system can be configured to determine and/or apply the power limit based at least in part on a parameter indicative of a flow rate of the flow of gases.

In a configuration, the power limit can be applied in response to the heater plate temperature exceeding a heater plate temperature threshold.

In a configuration, the system can be further configured to output a power to the conduit heating element, the power substantially not exceeding the power limit.

In a configuration, the system can be configured to determine and/or apply a power limit on the conduit heating element by controlling the conduit heating element voltage.

In a configuration, the system can be configured to determine and/or apply a power limit on the conduit heating element by controlling the conduit heating element current.

In a configuration, the system can be configured to determine and/or apply a power limit on the conduit heating element by controlling the conduit heating element resistance.

In a configuration, the power limit can be a limit on a duty cycle of the conduit heating element.

In a configuration, the system can be configured to determine and/or apply the power limit without requiring an electrical current input of the conduit heating element.

In a configuration, the system can be configured to determine and/or apply the power limit without requiring input from any combination of the following types of sensors: flow sensors, pressure sensors, humidity sensors, chamber inlet temperature sensors, chamber outlet temperature sensors, and/or patient end temperature sensors.

In a configuration, the system can be configured to determine and/or apply the power limit without requiring input from sensors downstream of the humidifier chamber.

In a configuration, the system may not comprise a sensor downstream of the humidifier chamber.

In a configuration, the system can be configured to determine and/or apply the power limit based solely on input from any combination of the following sensors: heater plate temperature, heater plate power, heating element power, and/or ambient temperature.

In a configuration, the system can be configured to determine and/or apply the power limit without requiring input from sensors in a gases flow pathway.

In a configuration, the system can be configured to determine and/or apply the power limit without requiring input from sensors located at or near a chamber outlet and/or a patient end.

In a configuration, the system may not comprise a sensor at or near the chamber outlet and/or the patient end.

In a configuration, applying the power limit may not comprise turning off the power to the conduit heating element and/or the one or more heater plate heating elements.

In a configuration, applying the power limit further can comprise turning off the power to the conduit heating element.

In a configuration, the power limit can be further configured to keep the user end enthalpy under a second enthalpy limit on the specific enthalpy for dry air, wherein the second enthalpy limit is higher than the first enthalpy limit.

In a configuration, the second enthalpy limit can be from 197 kJ/m$^3$ to 441 kJ/m$^3$.

In a configuration, the second enthalpy limit can be from 195 kJ/m$^3$ to 205 kJ/m$^3$.

In a configuration, the second enthalpy limit can be for redundancy over the first enthalpy limit.

In a configuration, the second enthalpy limit can be configured to protect the user from more severe harm than the first enthalpy limit.

In a configuration, the power limit can be further configured to keep the user end temperature under a second temperature limit, wherein the second temperature limit is higher than the first temperature limit.

In a configuration, the second temperature limit can be from 43°C to 63°C.

In a configuration, the second temperature limit can be from 43°C to 45°C.

In a configuration, the second temperature limit can be a second dew point temperature limit.

In a configuration, the second temperature limit can be for redundancy over the first temperature limit.

In a configuration, the second temperature limit can be configured to protect the user from more significant enthalpy level than the first temperature limit.

In a configuration, the system can be configured to apply the power limit to keep the user end temperature under the second temperature limit or the second enthalpy limit using a CPU or electronic circuitry outside of the CPU.

In a configuration, applying the power limit to keep the user end temperature or enthalpy under the second temperature limit or the second enthalpy limit can comprise turning off the power to the conduit heating element.

In a configuration, the power limit to keep the user end temperature or enthalpy under the second temperature limit or the second enthalpy limit can be applied based at least in part on the heater plate temperature.

In a configuration, the power limit to keep the user end temperature or enthalpy under the second temperature limit or the second enthalpy limit can be applied based at least in part on the ambient temperature.

In a configuration, the power limit to keep the user end temperature or enthalpy under the second temperature limit or the second enthalpy limit can decrease as the ambient temperature increases.

In a configuration, the power limit configured to keep the user end temperature or enthalpy under the second temperature limit or the second enthalpy limit can be applied only upon the heater plate temperature exceeding a second heater plate temperature threshold.

In a configuration, the power limit to keep the user end temperature or enthalpy under the second temperature limit or the second enthalpy limit can be applied by triggering a first protection circuit that comprises a comparator.

In a configuration, the power limit to keep the user end temperature or enthalpy under the second temperature limit or the second enthalpy limit can be based in part on a heater plate power.

In a configuration, the first protection circuit can apply the power limit based in part on the heater plate power.

In a configuration, the system can further a second protection circuit comprising a second comparator, the second protection circuit applying the power limit based in part on the heater plate power.

In a configuration, the system can comprise the inspiratory conduit.

In a configuration, the system can comprise an expiratory conduit.

An example respiratory or surgical humidifier system configured to deliver a flow of gases to a user, the system comprising: a base unit comprising: a heater plate including one or more heater plate heating elements; and a hardware controller configured to energize the one or more heating elements of the heater plate; and the base unit configured to receive a humidifier chamber including a thermally conductive base such that the thermally conductive base contacts the heater plate, the humidifier chamber configured to hold a volume of water, wherein the hardware controller is in electronic communication with a conduit heating element in an inspiratory conduit configured to transport gases from the humidifier chamber to a patient interface, the hardware controller programmed to determine and/or apply a power limit on the conduit heating element based at least in part on one or more parameters of the heater plate that includes a parameter indicative of a flow rate of the flow of gases.

In a configuration, determining and/or applying a power limit on the conduit heating element can comprise controlling power in the conduit heating element to, or below, the power limit.

In a configuration, determining and/or applying a power limit on the conduit heating element can comprise disabling a component of the system or the entire system if the power limit on the conduit heating element is exceeded.

In a configuration, disabling a component of the system can comprise disabling the conduit heating element.

In a configuration, disabling a component of the system can comprise disabling the heater plate.

In a configuration, the system can comprise a heater plate temperature sensor configured to measure a heater plate temperature and the one or more parameters of the heater plate, which hardware controller is programmed to determine and/or apply the power limit based at least in part on, includes at least the heater plate temperature.

In a configuration, the system can comprise an ambient temperature sensor configured to measure an ambient temperature and the hardware controller programmed to determine and/or apply a power limit on the conduit heating element based at least in part on the ambient temperature.

In a configuration, the power limit can be configured to keep a user end temperature of the gases under a temperature limit.

In a configuration, the temperature limit can be from 32°C to 45°C.

In a configuration, the temperature limit can be from 42°C to 45°C.

In a configuration, the temperature limit can be a dew point temperature limit.

In a configuration, the power limit can be configured to keep a user end enthalpy of the gases under a first enthalpy limit on a specific enthalpy for dry air.

In a configuration, the first enthalpy limit can be from 122 kJ/m$^3$ to 216 kJ/m$^3$.

In a configuration, the first enthalpy limit can be from 195 kJ/m$^3$ to 205 kJ/m$^3$.

In a configuration, the hardware controller can be programmed to determine and/or apply the power limit independent of control of energization of the one or more heating elements of the heater plate.

In a configuration, the one or more parameters of the heater plate, which the hardware controller is programmed to determine and/or apply the power limit can be based at least in part on, includes at least a heater plate power.

In a configuration, the hardware controller can be further programmed to apply the power limit in response to the heater plate temperature exceeding a heater plate temperature threshold.

In a configuration, the hardware controller can be further programmed to output a power to the conduit heating element, the power substantially not exceeding the power limit.

In a configuration, the hardware controller can be programmed to determine and/or apply a power limit on the conduit heating element by controlling the conduit heating element voltage.

In a configuration, the hardware controller can be programmed to determine and/or apply a power limit on the conduit heating element by controlling the conduit heating element current.

In a configuration, the hardware controller can be programmed to determine and/or apply a power limit on the conduit heating element by controlling the conduit heating element resistance.

In a configuration, the power limit can be a limit on a duty cycle of the conduit heating element.

In a configuration, the hardware controller can be programmed to determine and/or apply the power limit without requiring a current input of the conduit heating element.

In a configuration, the system can be configured to determine and/or apply the power limit without requiring input from sensors in a gases flow pathway.

In a configuration, the hardware controller can be programmed to determine and/or apply the power limit without requiring input from any combination of the following types of sensors: flow sensors, pressure sensors, humidity sensors, chamber inlet temperature sensors, chamber outlet temperature sensors, and/or patient end temperature sensors.

In a configuration, the hardware controller can be programmed to determine and/or apply the power limit without requiring input from sensors downstream of the humidifier chamber.

In a configuration, the system may not comprise a sensor downstream of the humidifier chamber.

In a configuration, the hardware controller can be programmed to determine and/or apply the power limit based solely on input from any combination of the following sensors: heater plate temperature, heater plate power, heating element power, and/or ambient temperature.

In a configuration, the hardware controller can be programmed to determine and/or apply the power limit without requiring input from sensors located at or near a chamber outlet and/or a patient end.

In a configuration, the system may not comprise a sensor at or near the chamber outlet and/or the patient end.

In a configuration, applying the power limit may not comprise turning off the power to the conduit heating element.

In a configuration, applying the power limit further comprises turning off the power to the conduit heating element and/or to the one or more heater plate heating elements.

In a configuration, the parameter indicative of the flow rate of the flow of gases can comprise a thermal conductivity value or a value indicative of a thermal conductivity between the heater plate and the flow of gases.

In a configuration, the power limit can comprise a power offset.

In a configuration, the power offset can be based at least in part on the parameter indicative of a flow rate of the flow of gases.

In a configuration, the power offset can be based at least in part on a difference between a threshold of the parameter indicative of a flow rate of the flow of gases and the parameter indicative of a flow rate of the flow of gases.

In a configuration, the power offset can be based at least in part on the heater plate temperature.

In a configuration, the power offset can be based at least in part on a difference between a heater plate temperature and a heater plate temperature threshold.

In a configuration, the power offset can be zero when the parameter indicative of a flow rate of the flow of gases is equal to or above the threshold of said parameter and the heater plate temperature is equal to or below the heater plate temperature threshold.

In a configuration, the power offset can be zero when a difference between the threshold of the parameter indicative of a flow rate of the flow of gases and the parameter indicative of a flow rate of the flow of gases is non-positive and/or the difference between the heater plate temperature and the heater plate temperature threshold is non-positive.

In a configuration, the power offset can be positive when the parameter indicative of a flow rate of the flow of gases is below the threshold of said parameter and the heater plate temperature is greater than the heater plate temperature threshold.

In a configuration, the power offset can be positive when the difference between the threshold of the parameter indicative of a flow rate of the flow of gases and the parameter indicative of a flow rate of the flow of gases is positive and the difference between the heater plate temperature and the heater plate temperature threshold is positive.

In a configuration, the system can comprise the inspiratory conduit.

In a configuration, the system can comprise an expiratory conduit.

An example respiratory or surgical humidifier system configured to deliver a flow of gases to a user, the system comprising: a base unit comprising: a heater plate including one or more heater plate heating elements; a heater plate temperature sensor configured to measure a heater plate temperature; an ambient temperature sensor configured to measure an ambient temperature; and a hardware controller configured to energize the one or more heating elements of the heater plate; and the base unit configured to receive a humidifier chamber including a thermally conductive base such that the thermally conductive base contacts the heater plate, the humidifier chamber configured to hold a volume of water, wherein the hardware controller is programmed to determine a reduction in a flow rate of the gases based at least in part on one or more parameters of the heater plate.

In a configuration, the hardware controller can be programmed to determine the reduction in the flow rate without calculating a flow rate value.

In a configuration, the system may not comprise a flow sensor.

In a configuration, the hardware controller can be programmed to determine the reduction in the flow rate of the gases based at least in part on a heater plate power.

In a configuration, the hardware controller can be programmed to determine a reduction in the flow rate of the gases based at least in part on the heater plate temperature and/or the ambient temperature.

In a configuration, the hardware controller can be programmed to limit a power to a conduit heating element of an inspiratory conduit when the flow of gases is in a low flow state and the heater plate temperature exceeds a threshold, the inspiratory conduit configured to transport the gases from the humidifier chamber to a patient interface.

In a configuration, the system can comprise an inspiratory conduit.

In a configuration, the system can comprise an expiratory conduit.

An example respiratory or surgical humidifier system configured to deliver a flow of gases to a user, the system comprising: a base unit comprising: a heater plate including one or more heater plate heating elements; and a hardware controller configured to energize the one or more heating elements of the heater plate, the base unit configured to receive a humidifier chamber including a thermally conductive base such that the thermally conductive base contacts the heater plate, the humidifier chamber configured to hold a volume of water, wherein the hardware controller is configured to be in electronic communication with a conduit heating element in an inspiratory conduit configured to transport the gases from the humidifier chamber to a patient interface, the system configured to determine and/or apply a power limit on the conduit heating element based at least in part on a heater plate temperature and/or a heater plate power.

In a configuration, determining and/or applying a power limit on the conduit heating element can comprise controlling power in the conduit heating element to, or below, the power limit.

In a configuration, determining and/or applying a power limit on the conduit heating element can comprise disabling a component of the system or the entire system if the power limit on the conduit heating element is exceeded.

In a configuration, disabling a component of the system can comprise disabling the conduit heating element.

In a configuration, disabling a component of the system can comprise disabling the heater plate.

In a configuration, the system can further comprise a heater plate temperature sensor configured to measure a heater plate temperature and the system is configured to determine and/or apply the power limit based at least in part on the heater plate temperature.

In a configuration, the system can further comprise an ambient temperature sensor configured to measure an ambient temperature and the system configured to determine and/or apply a power limit on the conduit heating element based at least in part on the ambient temperature.

In a configuration, the system can further comprise one or more thermistors.

In a configuration, the one or more thermistors comprise at least one negative temperature coefficient (NTC) thermistor.

In a configuration, the system can be configured to apply the power limit using electronic circuitry outside the hardware controller.

In a configuration, the hardware controller can comprise a CPU.

In a configuration, the system can be configured to determine and/or apply a power limit on the conduit heating element by controlling the conduit heating element voltage.

In a configuration, the system can be configured to determine and/or apply a power limit on the conduit heating element by controlling the conduit heating element current.

In a configuration, the system can be configured to determine and/or apply a power limit on the conduit heating element by controlling the conduit heating element resistance.

In a configuration, a power limit can comprise a limit on a duty cycle.

In a configuration, the power limit can be configured to keep a user end temperature of the gases under a temperature limit.

In a configuration, the temperature limit can be from 43°C to 63°C.

In a configuration, the temperature limit can be from 43°C to 45°C.

In a configuration, the temperature limit can be a dew point temperature limit.

In a configuration, the power limit can be configured to keep the user end enthalpy under an enthalpy limit on a specific enthalpy for dry air.

In a configuration, the enthalpy limit can be from 197 $kJ/m^3$ to 441 $kJ/m^3$.

In a configuration, the enthalpy limit can be from 195 $kJ/m^3$ to 205 $kJ/m^3$.

In a configuration, the power limit can be for redundancy over a heater plate software and/or hardware safety feature.

In a configuration, the power limit can be configured to protect the user from more significant enthalpy level than the heater plate software and/or hardware safety feature.

In a configuration, the system configured to determine and/or apply a power limit on the conduit heating element can be based at least in part on a parameter indicative of a flow rate of the flow of gases.

In a configuration, the power limit can decrease as the ambient temperature increases.

In a configuration, the power limit can be determined and/or apply in response to the heater plate temperature exceeding a temperature threshold.

In a configuration, the power limit can be applied by triggering a first protection circuit that comprises a comparator.

In a configuration, the first protection circuit can trigger the power limit based at least in part on a heater plate power.

In a configuration, the first protection circuit can trigger the power limit based at least in part on the heater plate power.

In a configuration, the system further can comprise a second protection circuit comprising a second comparator, the second protection circuit configured to apply the power limit based at least in part on the heater plate power.

In a configuration, the system can be configured to determine and/or apply the power limit without requiring input from any combination of the following types of sensors: flow sensors, pressure sensors, humidity sensors, chamber inlet temperature sensors, chamber outlet temperature sensors, and/or patient end temperature sensors.

In a configuration, the system can be configured to determine and/or apply the power limit without requiring input from sensors downstream of the humidifier chamber.

In a configuration, the system may not comprise a sensor downstream of the humidifier chamber.

In a configuration, the system can be configured to determine and/or apply the power limit based solely on input from any combination of the following sensors: heater plate temperature, heater plate power, heating element power, and/or ambient temperature.

In a configuration, the system can be configured to determine and/or apply the power limit without requiring input from sensors in the gases flow pathway.

In a configuration, the system can be configured to determine and/or apply the power limit without requiring input from sensors located at or near a chamber outlet and/or a patient end.

In a configuration, the system may not comprise a sensor at or near the chamber outlet and/or the patient end.

In a configuration, the system comprises the inspiratory conduit.

In a configuration, the system comprises an expiratory conduit.

An example respiratory or surgical humidifier system configured to deliver a flow of gases to a user, the system comprising: a base unit comprising: a heater plate including one or more heater plate heating elements; a hardware controller configured to energize the one or more heating elements of the heater plate, the base unit configured to receive a humidifier chamber including a thermally conductive base such that the thermally conductive base contacts the heater plate, the humidifier chamber configured to hold a volume of water, wherein the hardware controller is configured to be in electronic communication with a conduit heating element in an inspiratory conduit configured to transport the gases from the humidifier chamber to a patient interface; and an over-enthalpy protection circuit configured to apply a power limit on a conduit heating element power based at least in part on a heater plate temperature and/or a heater plate power, the over-enthalpy protection circuit comprising a comparator.

In a configuration, applying a power limit on the conduit heating element can comprise controlling power in the conduit heating element to, or below, the power limit.

In a configuration, applying a power limit on the conduit heating element can comprise disabling a component of the system or the entire system if the power limit on the conduit heating element is exceeded.

In a configuration, disabling a component of the system can comprise disabling the conduit heating element.

In a configuration, disabling a component of the system can comprise disabling the heater plate.

In a configuration, the system can comprise a heater plate temperature sensor configured to measure a heater plate temperature and the over-enthalpy protection circuit is configured to apply the power limit based at least in part on the heater plate temperature.

In a configuration, the system can comprise an ambient temperature sensor configured to measure an ambient temperature and the over-enthalpy protection circuit is configured to apply a power limit on a conduit heating element power based at least in part on the ambient temperature.

In a configuration, the system can comprise one or more thermistors.

In a configuration, the one or more thermistors can comprise at least one negative temperature coefficient (NTC) thermistor.

In a configuration, the over-enthalpy protection circuit can be configured to determine and/or apply a power limit on the conduit heating element by controlling the conduit heating element voltage.

In a configuration, the over-enthalpy protection circuit can be configured to determine and/or apply a power limit on the conduit heating element by controlling the conduit heating element current.

In a configuration, the system can be over-enthalpy protection circuit to determine and/or apply a power limit on the conduit heating element by controlling the conduit heating element resistance.

In a configuration, the power limit can comprise limiting a duty cycle of the conduit heating element.

In a configuration, the over-enthalpy protection circuit can be configured to keep a user end temperature of the gases under a temperature limit.

In a configuration, the temperature limit can be from 43°C to 63°C.

In a configuration, the temperature limit can be from 43°C to 45°C.

In a configuration, the temperature limit can be a dew point temperature limit.

In a configuration, the temperature limit can be a dew point temperature limit.

In a configuration, the power limit can be configured to keep the user end enthalpy under an enthalpy limit.

In a configuration, the enthalpy limit can be from 195 kJ/m$^3$ to 441 kJ/m$^3$.

In a configuration, the enthalpy limit can be from 195 kJ/m$^3$ to 205 kJ/m$^3$.

In a configuration, the over-enthalpy protection circuit can be for redundancy over a heater plate software and/or hardware safety feature.

In a configuration, the power limit can be configured to protect the user from more significant enthalpy level than the software conduit heating element safety.

In a configuration, the power limit can decrease as the ambient temperature increases.

In a configuration, the over-enthalpy protection circuit can be configured to apply the power limit only upon the heater plate temperature exceeding a temperature threshold.

In a configuration, the system can comprise a second over-enthalpy protection circuit including a second comparator.

In a configuration, the second over-enthalpy protection circuit is configured to apply the power limit based at least in part on the conduit heating element power.

In a configuration, the second over-enthalpy protection circuit can be configured to apply the power limit based at least in part on the heater plate power.

In a configuration, the second over-enthalpy protection circuit can be configured to apply the power limit based at least in part on the heater plate temperature.

In a configuration, the second over-enthalpy protection circuit can be configured to apply the power limit based at least in part on the ambient temperature.

In a configuration, the system can be configured to determine and/or apply the power limit without requiring input from any combination of the following types of sensors: flow sensors, pressure sensors, humidity sensors, chamber inlet temperature sensors, chamber outlet temperature sensors, and/or patient end temperature sensors.

In a configuration, the system can be configured to determine and/or apply the power limit without requiring input from sensors downstream of the humidifier chamber.

In a configuration, the system may not comprise a sensor downstream of the humidifier chamber.

In a configuration, the system can be configured to determine and/or apply the power limit based solely on input from any combination of the following sensors: heater plate temperature, heater plate power, heating element power, and/or ambient temperature.

In a configuration, the system can be configured to determine and/or apply the power limit without requiring input from sensors in a gases flow pathway.

In a configuration, the system can be configured to determine and/or apply the power limit without requiring input from sensors located at or near a chamber outlet and/or a patient end.

In a configuration, the system may not comprise a sensor at or near the chamber outlet and/or the patient end.

In a configuration, the system can comprise the inspiratory conduit.

In a configuration, the system can comprise an expiratory conduit.

In a configuration, the power limit on the conduit heating element can be applied when the flow of gases is at below 7 L/min.

An example respiratory or surgical humidifier system configured to deliver a flow of gases to a user, the system comprising: a base unit comprising: a heater plate including one or more heater plate heating elements; and a hardware controller configured to energize the one or more heating elements of the heater plate, the base unit configured to receive a humidifier chamber including a thermally conductive base such that the thermally conductive base contacts the heater plate, the humidifier chamber configured to hold a volume of water, wherein the hardware controller is configured to be in electronic communication with a conduit heating element in an inspiratory conduit configured to transport the gases from the humidifier chamber to a patient interface, the system configured to determine and/or apply a first power limit and a second power limit on the conduit heating element based at least in part on one or more parameters of the heater plate.

In a configuration, the first power limit is controlling to, or below, a power limit.

In a configuration, the second power limit is disabling power to the conduit heating element.

In a configuration, a threshold to enact the first power limit is lower than a threshold to enact the second power limit.

In a configuration, the first power limit corresponds with a lower patient end enthalpy limit than the second power limit.

According to the invention, the first power limit is applied by the hardware controller and the second power limit is applied by a protection circuit.

In a configuration, determining and/or applying a first power limit and/or a second power limit on the conduit heating element comprises controlling power in the conduit heating element to the appl the first power limit and/or the second power limit.

In a configuration, determining and/or applying a first power limit and a second power limit on the conduit heating element can comprise disabling a component of the system or the entire system if the first power limit and/or the second power limit on the conduit heating element is exceeded.

In a configuration, disabling a component of the system comprises disabling the conduit heating element.

In a configuration, disabling a component of the system comprises disabling the heater plate.

In a configuration, the system can further comprise a heater plate temperature sensor configured to measure a heater plate temperature and the one or more parameters of the heater plate, which the system is configured to determine and/or apply the first power limit and/or the second power limit based at least in part on, includes at least the conduit heating element power.

In a configuration, the system can further comprise a heater plate temperature sensor configured to measure a heater plate temperature and the one or more parameters of the heater plate, which the system is configured to determine and/or apply the first power limit and/or the second power limit based at least in part on, includes at least the heater plate temperature.

In a configuration, the system can further comprise an ambient temperature sensor configured to measure an ambient temperature and the system is configured to determine and/or apply a applying a first power limit and/or a second power limit on the conduit heating element based at least in part on the ambient temperature.

In a configuration, the heater plate temperature sensor and/or the ambient temperature sensor can comprise one or more thermistors.

In a configuration, the one or more thermistors comprise at least one negative temperature coefficient (NTC) thermistor.

In a configuration, the one or more parameters of the heater plate, which the system is configured to determine and/or apply the first power limit and/or the second power limit based at least in part on, includes at least a heater plate power.

In a configuration, heater plate power is controlled by heater plate voltage.

In a configuration, the first power limit and/or the second power limit on the conduit heating element is non-binary.

In a configuration, the first power limit and/or the second power limit is configured to keep a user end temperature of the gases under a first temperature limit.

In a configuration, the first temperature limit is from 32°C to 45°C.

In a configuration, the first temperature limit is 40°C.

In a configuration, the first temperature limit is a first dew point temperature limit.

In a configuration, the first power limit and/or the second power limit is configured to keep a user end enthalpy of the gases under a first enthalpy limit on a specific enthalpy for dry air.

In a configuration, the first enthalpy limit is from 122 kJ/m$^3$ to 216 kJ/m$^3$.

In a configuration, the first enthalpy limit is from 195 kJ/m$^3$ to 205 kJ/m$^3$.

In a configuration, the system is configured to determine and/or apply the first power limit and/or the second power limit independent of control of energization of the one or more heating elements of the heater plate.

In a configuration, the system is configured to determine and/or apply the first power limit and/or the second power limit using the hardware controller.

In a configuration, the hardware controller comprises a central processing unit (CPU).

In a configuration, the system is configured to determine and/or apply the first power limit and/or the second power limit based at least in part on a parameter indicative of a flow rate of the flow of gases.

In a configuration, the first power limit and/or the second power limit is applied in response to the heater plate temperature exceeds a heater plate temperature threshold.

In a configuration, the system is further configured to output a power to the conduit heating element, the power substantially not exceeding a first power limit and/or a second power limit.

In a configuration, the system is configured to determine and/or apply a first power limit and/or a second power limit on the conduit heating element by controlling the conduit heating element voltage.

In a configuration, the system is configured to determine and/or apply a first power limit and/or a second power limit on the conduit heating element by controlling the conduit heating element current.

In a configuration, the system is configured to determine and/or apply a first power limit and/or a second power limit on the conduit heating element by controlling the conduit heating element resistance.

In a configuration, the first power limit and/or the second power limit is a limit on a duty cycle of the conduit heating element.

In a configuration, the system is configured to determine and/or apply the first power limit and/or the second power limit without requiring an electrical current input of the conduit heating element.

In a configuration, the system is configured to determine and/or apply the first power limit and/or the second power limit without requiring input from any combination of the following types of sensors: flow sensors, pressure sensors, humidity sensors, chamber inlet temperature sensors, chamber outlet temperature sensors, and/or patient end temperature sensors.

In a configuration, the system is configured to determine and/or apply the first power limit and/or the second power limit without requiring input from sensors downstream of the humidifier chamber.

In a configuration, the system does not comprise a sensor downstream of the humidifier chamber.

In a configuration, the system is configured to determine and/or apply the first power limit and/or the second power limit based solely on input from any combination of the following sensors: heater plate temperature, heater plate power, heating element power, and/or ambient temperature.

In a configuration, the system is configured to determine and/or apply the first power limit and/or the second power limit without requiring input from sensors in a gases flow pathway.

In a configuration, the system is configured to determine and/or apply the first power limit and/or the second power limit without requiring input from sensors located at or near a chamber outlet and/or a patient end.

In a configuration, the system does not comprise a sensor at or near the chamber outlet and/or the patient end.

In a configuration, applying the first power limit and/or the second power limit does not comprise turning off the power to the conduit heating element and/or the one or more heater plate heating elements.

In a configuration, applying the first power limit and/or the second power limit further comprises turning off the power to the conduit heating element.

In a configuration, the first power limit and/or the second power limit is further configured to keep the user end enthalpy under a second enthalpy limit on the specific enthalpy for dry air, wherein the second enthalpy limit is higher than the first enthalpy limit.

In a configuration, the second enthalpy limit is from 197 kJ/m$^3$ to 441 kJ/m$^3$.

In a configuration, the second enthalpy limit is from 195 kJ/m$^3$ to 205 kJ/m$^3$.

In a configuration, the second enthalpy limit is for redundancy over the first enthalpy limit.

In a configuration, the second enthalpy limit is configured to protect the user from more severe harm than the first enthalpy limit.

In a configuration, the first power limit and/or the second power limit is further configured to keep the user end temperature under a second temperature limit, wherein the second temperature limit is higher than the first temperature limit.

In a configuration, the second temperature limit is from 43°C to 63°C.

In a configuration, the second temperature limit is from 43°C to 45°C.

In a configuration, the second temperature limit is a second dew point temperature limit.

In a configuration, the second temperature limit is for redundancy over the first temperature limit.

In a configuration, the second temperature limit is configured to protect the user from more significant enthalpy level than the first temperature limit.

In a configuration, the system is configured to apply the first power limit and/or the second power limit to keep the user end temperature under the second temperature limit or the second enthalpy limit using a CPU or electronic circuitry outside of the CPU.

In a configuration, applying the first power limit and/or the second power limit to keep the user end temperature or enthalpy under the second temperature limit or the second enthalpy limit comprises turning off the power to the conduit heating element.

In a configuration, the first power limit and/or the second power limit to keep the user end temperature or enthalpy under the second temperature limit or the second enthalpy limit is applied based at least in part on the heater plate temperature.

In a configuration, the first power limit and/or the second power limit to keep the user end temperature or enthalpy under the second temperature limit or the second enthalpy limit is applied based at least in part on the ambient temperature.

In a configuration, the first power limit and/or the second power limit to keep the user end temperature or enthalpy under the second temperature limit or the second enthalpy limit decreases as the ambient temperature increases.

In a configuration, the first power limit and/or the second power limit configured to keep the user end temperature or enthalpy under the second temperature limit or the second enthalpy limit is applied only upon the heater plate temperature exceeding a second heater plate temperature threshold.

In a configuration, the first power limit and/or the second power limit to keep the user end temperature or enthalpy under the second temperature limit or the second enthalpy limit is applied by triggering a first protection circuit that comprises a comparator.

In a configuration, the first power limit and/or the second power limit to keep the user end temperature or enthalpy under the second temperature limit or the second enthalpy limit is based in part on a heater plate power.

In a configuration, the first protection circuit applies the first power limit and/or the second power limit based in part on the heater plate power.

In a configuration, a second protection circuit comprising a second comparator, the second protection circuit applying the first power limit and/or the second power limit based in part on the heater plate power.

In a configuration, the system can comprise the inspiratory conduit.

In a configuration, the system can comprise an expiratory conduit.

## BRIEF DESCRIPTION OF THE DRAWINGS

These and other features, aspects, and advantages of the present disclosure are described with reference to the drawings of certain configurations, which are intended to schematically illustrate certain configurations and not to limit the disclosure.

Throughout the drawings, labels such as "heater wire" or "HW" illustrate an example heating element of a conduit (for example an inspiratory or insufflation conduit) of a humidifier system and are not limiting. Additionally, labels such as "duty" or "duty cycle" illustrate an example type of power control to a heater plate or a heating element of an inspiratory conduit of a humidifier system and are not limiting.

Figure 1A illustrates an example respiratory humidifier system.
Figure 1B illustrates an example heater base unit of the respiratory humidifier system of Figure 1A.

Figure 1C illustrates a partial view of the heater base unit and an example inspiratory conduit heating element adapter of Figure 1A.

Figure 2A illustrates schematically an example respiratory humidifier system with separate blower and heater base units and connected to a nasal mask.

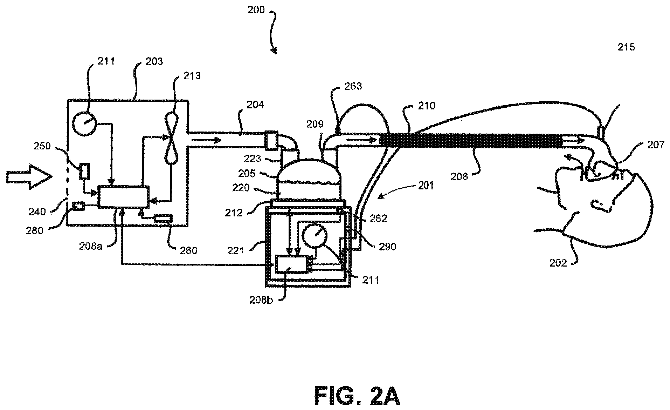

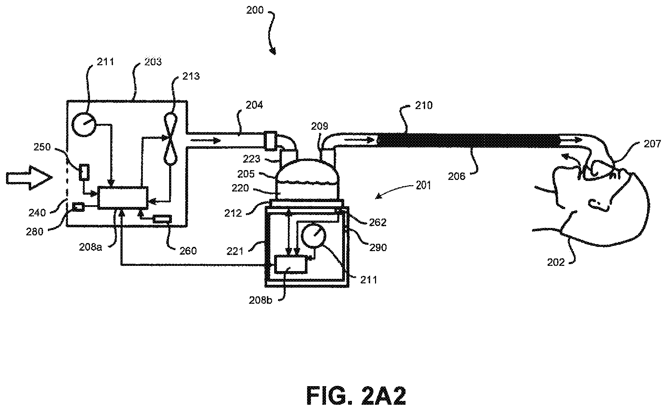

Figure 2A2 illustrates schematically the respiratory humidifier system of Figure 2A without certain sensors.

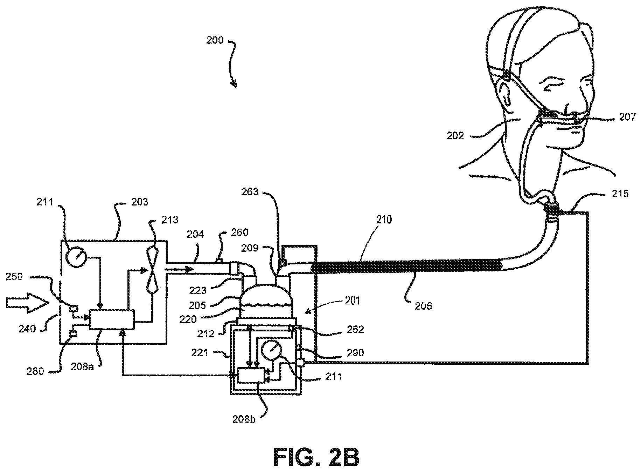

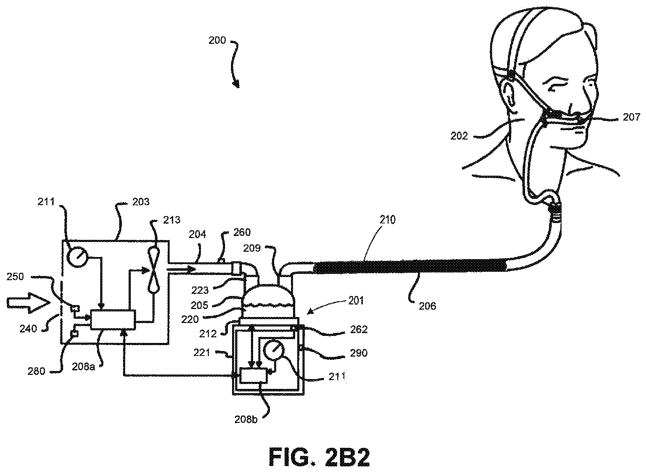

Figure 2B illustrates schematically the respiratory humidifier system of Figure 2A connected to a nasal cannula.

Figure 2B2 illustrates schematically the respiratory humidifier system of Figure 2B without certain sensors.

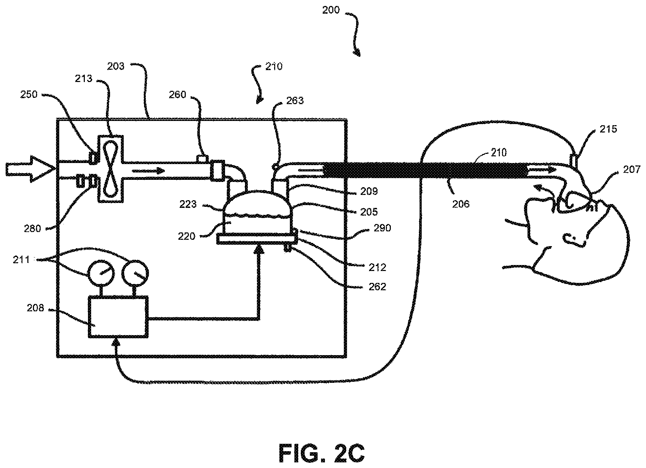

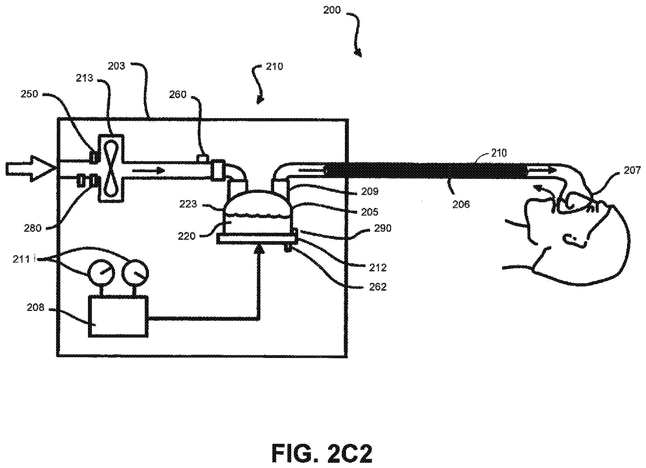

Figure 2C illustrates schematically an example respiratory humidifier system with an integrated blower and heater base unit.

Figure 2C2 illustrates schematically the respiratory humidifier system of Figure 2C without certain sensors.

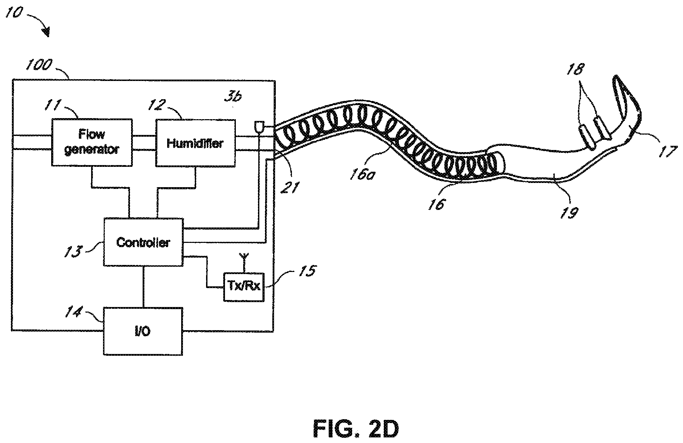

Figure 2D illustrates an example high flow therapy system.

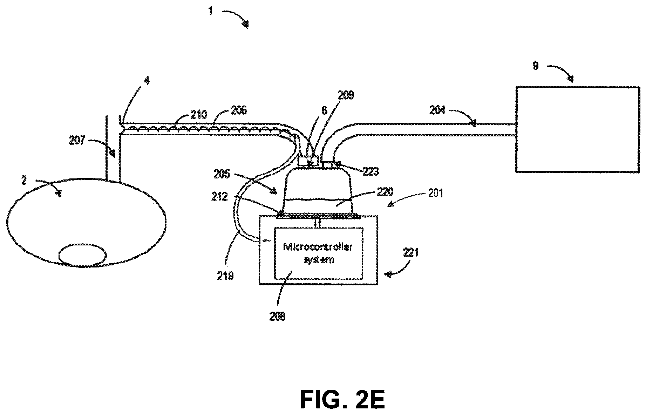

Figure 2E illustrates an example surgical system.

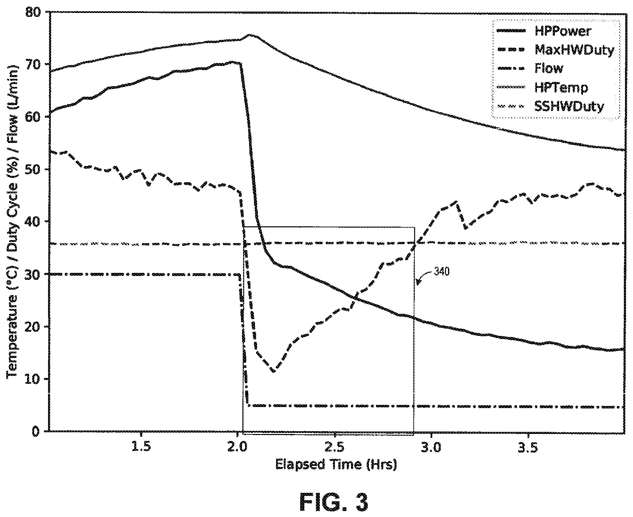

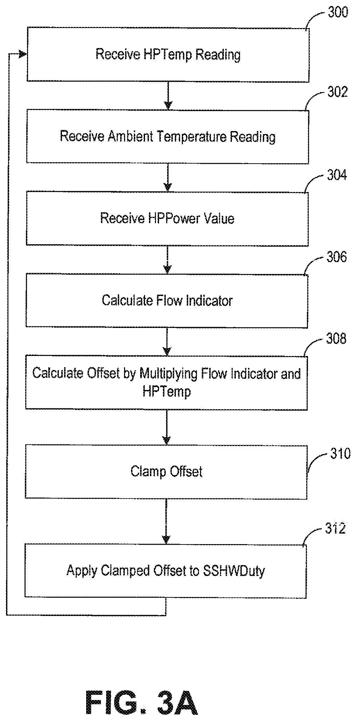

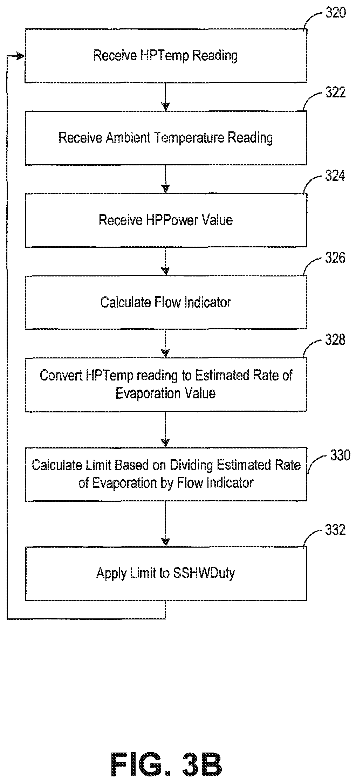

Figure 3 illustrates a graph showing certain parameters of a respiratory humidifier system during a large flow rate drop.

Figure 3A illustrates an example process of determining and/or applying an over-enthalpy protection by a hardware controller.

Figure 3B illustrates another example process of determining and/or applying an over-enthalpy protection by a hardware controller.

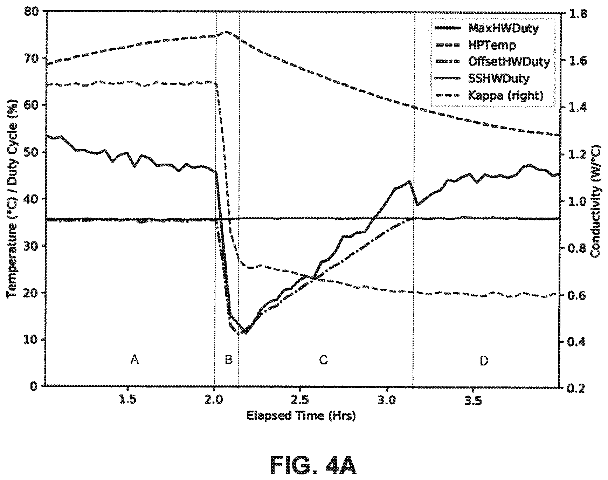

Figures 4A-4B illustrate example parameters of the operation of a humidifier system during an example over-enthalpy scenario when the processes of Figures 3A-B are implemented.

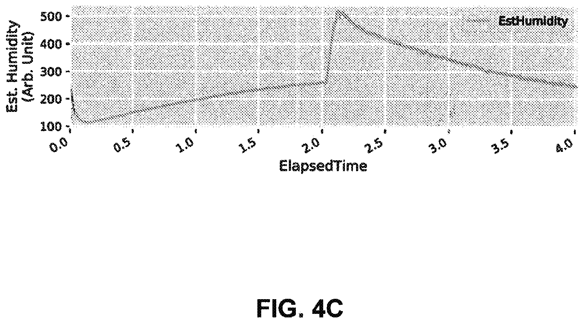

Figure 4C illustrates a plot of an estimated humidity at the chamber outlet during a large flow drop event.

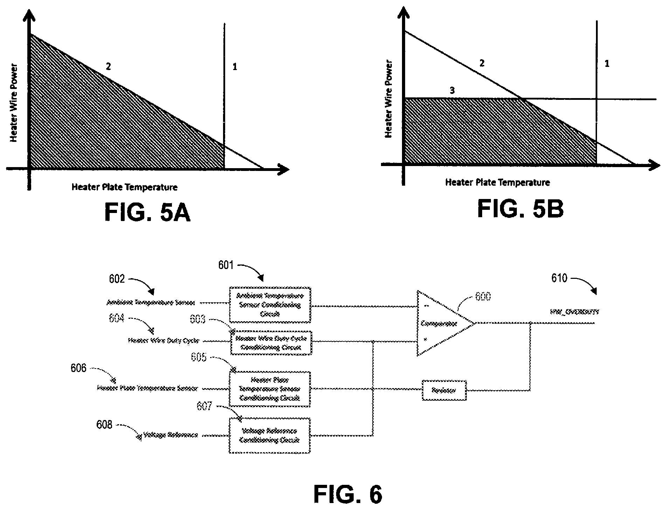

Figures 5A and 5B illustrate principles of limiting power to the heating element of the inspiratory conduit based on steady state conditions.

Figure 6 illustrates an example over-enthalpy protection circuit based in part on a heater plate temperature input and an ambient temperature input.

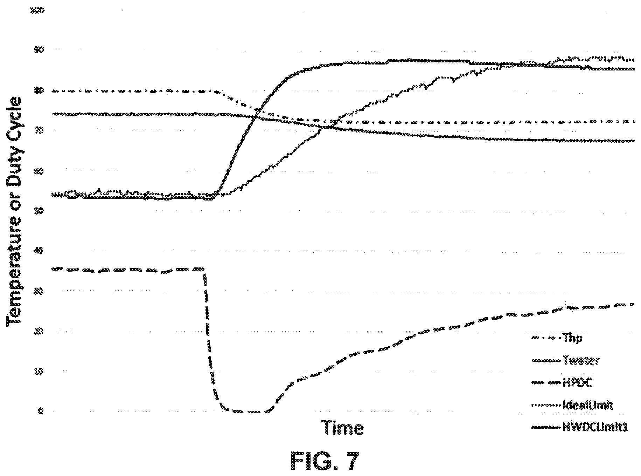

Figure 7 illustrates parameters of a humidifier system under transient heater plate temperature conditions, including differences between the heater plate temperature (Thp) and the water temperature (Twater).

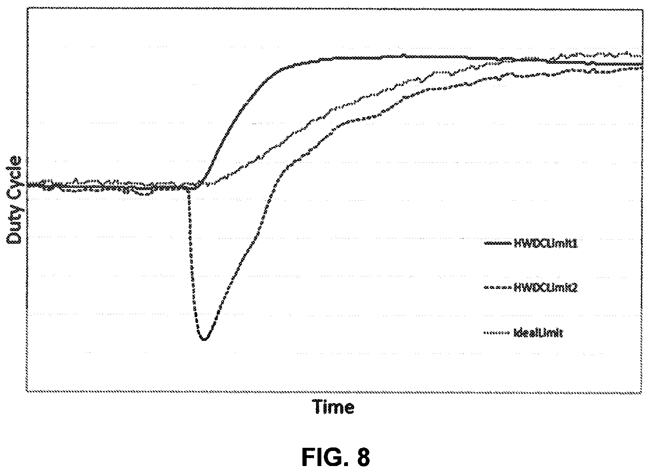

Figure 8 illustrates a power limit on the heating element of the inspiratory conduit by over-enthalpy protection based on steady state conditions (HWDCLimit1) and a power limit on the heating element of the inspiratory conduit by over-enthalpy protection based on an estimation of the water temperature (HWDCLimit2).

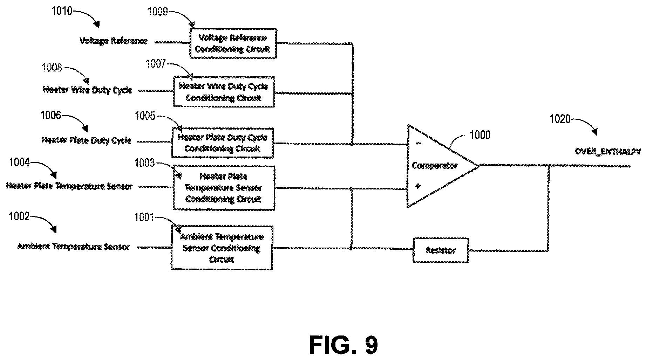

Figure 9 illustrates an example over-enthalpy protection circuit based in part on the heater plate power input.

DETAILED DESCRIPTION

Although certain configurations and examples are described below, those of skill in the art will appreciate that the disclosure extends beyond the specifically disclosed configurations and/or uses and obvious modifications and equivalents thereof. Thus, it is intended that the scope of the disclosure herein disclosed should not be limited by any particular configurations described below. For example, the temperature and/or enthalpy parameters provided in the present disclosure are examples and should not be limiting.

The present disclosure provides examples of a respiratory or surgical humidifier system configured to supply humidified and/or heated gases to a patient or user in multiple modes. The modes for the respiratory humidifier can include at least an invasive mode (for example, for patients with a bypassed airway) and a non-invasive mode (for example, for patients or users with breathing masks). Each mode can have a number of humidity settings, which can be expressed as a dew point or absolute humidity. The respiratory humidifier is controlled to deliver, at an outlet of the humidification chamber and/or the patient end of the inspiratory conduit, humidified gases having a dew point (or absolute humidity) at or near a predetermined humidity level. For example, a user can select a setting appropriate for the current mode of operation. A number of humidity settings may be provided. For example, the humidity settings may be equivalent to a dew point of 37°C, 31°C, 29°C, 27°C, or others. The humidity setting equivalent to a dew point of 37°C may be suitable for invasive therapy (that is, where the patient's upper airways are bypassed) whereas the other humidity settings may be suitable for non-invasive therapy, although the humidity settings may not be restricted to a particular type of therapy. Alternatively, each humidity setting may be continuously variable between upper and lower limits. A lower humidity setting may be selected by the user to reduce condensation or "rain-out" in the inspiratory conduit, or a higher humidity setting may be selected to improve patient comfort or physiological benefits. Some respiratory humidifier systems disclosed herein can also include a high flow, unsealed mode, or any other modes known to those of skill in the art. The disclosed circuits and methods may be similarly applied in a surgical humidifier which may be used, for example, in laparoscopic surgery or open surgery.

An example of a high flow, unsealed mode (herein referred to as Optiflow® mode) is marketed as Optiflow® by Fisher and Paykel Healthcare Limited of Auckland, New Zealand. High flow therapy as discussed herein is intended to be given its typical ordinary meaning, as understood by a person of skill in the art, which generally refers to a respiratory assistance system delivering a targeted flow of humidified respiratory gases via an intentionally unsealed patient interface with flow rates generally intended to meet or exceed inspiratory flow of a user. Typical patient interfaces include, but are not limited to, a nasal or tracheal patient interface. Typical flow rates for adults often range from, but are not limited to, about fifteen liters per minute to about sixty liters per minute or greater. Typical flow rates for pediatric users (such as neonates, infants and children) often range from, but are not limited to, about one liter per minute per kilogram of user weight to about three liters per minute per kilogram of user weight or greater. High flow therapy can also optionally include gas mixture compositions including supplemental oxygen and/or administration of therapeutic medicaments. High flow therapy is often referred to as nasal high flow (NHF), humidified high flow nasal cannula (HHFNC), high flow nasal oxygen (HFNO), high flow therapy (HFT), or tracheal high flow (THF), among other common names. For example, in some configurations, for an adult user "high flow therapy" may refer to the delivery of gases to a user at a flow rate of greater than or equal to about 10 litres per minute (10 LPM), such as between about 10 LPM and about 100 LPM, or between about 15 LPM and about 95 LPM, or between about 20 LPM and about 90 LPM, or between about 25 LPM and about 85 LPM, or between about 30 LPM and about 80 LPM, or between about 35 LPM and about 75 LPM, or between about 40 LPM and about 70 LPM, or between about 45 LPM and about 65 LPM, or between about 50 LPM and about 60 LPM. In some configurations, for a neonatal, infant, or child user 'high flow therapy' may refer to the delivery of gases to a user at a flow rate of greater than 1 LPM, such as between about 1 LPM and about 25 LPM, or between about 2 LPM and about 25 LPM, or between about 2 LPM and about 5 LPM, or between about 5 LPM and about 25 LPM, or between about 5 LPM and about 10 LPM, or between about 10 LPM and about 25 LPM, or between about 10 LPM and about 20 LPM, or between about 10 LPM and 15 LPM, or between about 20 LPM and 25 LPM. A high flow therapy apparatus with an adult user, a neonatal, infant, or child user, may deliver gases to the user at a flow rate of between about 1 LPM and about 100 LPM, or at a flow rate in any of the sub-ranges outlined above.

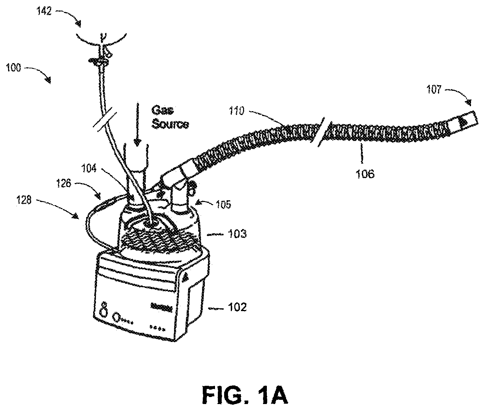

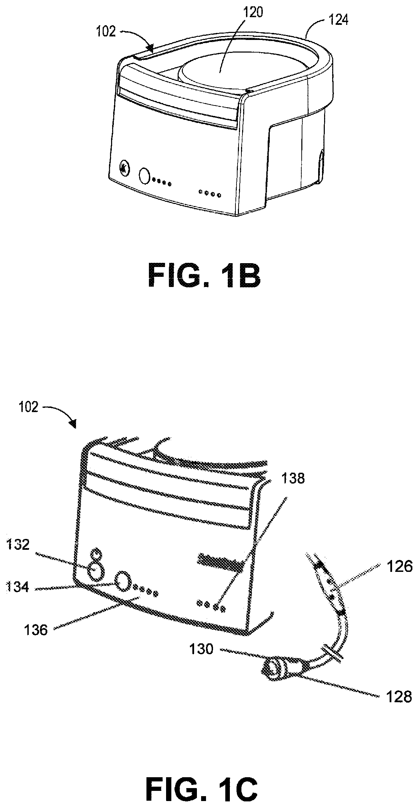

Referring to Figure 1A, an example respiratory humidifying system 100 can include a heater base unit 102 having a heater plate 120 (see Figure 1B). The heater plate 120 can include one or more heating element(s). The heater base unit 102 can have a housing and a controller (for example, a microprocessor, which can include a central processing unit (CPU)) contained within the housing for controlling the energization of the heating element(s) of the heater plate 120.

The heater plate 120 can include a temperature sensor (see temperature sensor 262 in Figure 2A) (for example, a temperature transducer, thermistor, or other types of temperature sensor). The temperature sensor can be a negative temperature coefficient (NTC) thermistor, or a positive temperature coefficient (PTC) thermistor. Multiple and optionally different temperature sensors (for example, two temperature sensors) may also be used. The temperature sensor(s) can measure a temperature of the heater plate 120. The temperature sensor(s) can be in electrical communication with the controller in the heater base unit 102 so that the controller can monitor the temperature of the heater plate 120. Measurements made by the temperature sensor(s) can be used as an input for over-enthalpy protection that will be described below.

The temperature sensor can also optionally include two or more thermistors. Each thermistor can act as a voltage divider. An average of the readings from the two thermistors can be used as the heater plate temperature. Two or more thermistors may also be used for redundancy. Additional thermistors can also be included. The temperature sensor can be positioned on an underside surface of the heater plate. The temperature sensors can be indirectly secured to the underside surface of the heater plate such that the temperature sensors are spaced apart from the underside surface of the heater plate. For example, the temperature sensors can be preferably placed under a top plate of the heater plate 120. The top plate is the plate that is exposed and is positioned to be in contact with the base of a humidifier chamber when the humidifier chamber is positioned in an operative position on the heater base unit. The temperature sensors can be positioned at an edge of the heater plate 120 or substantially in the center of the heater plate 120. A water temperature sensor can also be included. The water temperature sensor can be in addition to the heater plate temperature sensor or an alternative to the heater plate temperature sensor. The heating element(s) of the heater plate can include nichrome wire or other types of heating element(s) wrapped around an electrical insulator block or core. The heater plate 120 can include a plurality of electrical insulation layers. The heater plate 120 can include a bottom plate with the multiple parts being screwed or bolted together. Alternatively, the heater plate 120 can include a plurality of layers that can be laminated together or be adhered together to form a unitary heater plate. The heating element(s) of the heater plate 120 can alternatively optionally be formed as a metal layer on an insulating substrate by etching, or deposition, or any suitable arrangement.

The humidifier chamber 103 can be removably received and retained on the heater base unit 102, such that the humidifier chamber base is positioned in contact with the heater plate 120 in the heater base unit 102. Referring to Figure 1B, which illustrates an example of the heater base unit 102 of Figure 1A, the humidifying base unit 102 can have a collar 124 for engaging with a flange on the humidifier chamber 103. The collar 124 defines a lip that engages the flange of the humidifier chamber 103 to retain the humidifier chamber 103 in an operative position on the heater base unit 102. The humidifier chamber 103 can include a thermally conductive base. When engaged with the heater base unit 102, the conductive base of the humidifier chamber 103 can be in contact with the heater plate 120, such as an upper surface of the top plate of the heater plate 120. Water inside the humidifier chamber 103 is heated when a power signal is sent to the heating element to energize the heating element of the heater plate 120. The humidifier chamber 103 can also be connected to a water source 142, which can add water to the humidifier chamber 103 when the water is low or completely out in the humidifier chamber 103. Adding of water to the humidifier chamber 103 can be manually performed or controlled by the controller, such as upon a warning from the system 100 that there may be a low water or water-out condition and/or based on at least one float in the chamber 103. The controller can control a valve system to add water into the humidification chamber 103.

With continued reference to Figure 1A, the gases to be humidified can include one or more of air, oxygen, anesthetic, other auxiliary gases, or any mixture of gases. The gases can be supplied to the humidifier chamber 103 through a gases inlet 104, which can be connected to a gases source, such as a ventilator, or in the case of CPAP therapy a CPAP blower, or a remote source. For high flow therapy, a blower or further alternatively a wall source with a flow and/or pressure regulator can supply the gases. The humidifier chamber 103 also includes a gases outlet 105, which can connect to an inspiratory conduit 106. The inspiratory conduit 106 can convey humidified and heated gases to a user. A user end 107 of the inspiratory conduit 106 can connect to a patient interface, such as a nasal cannula or a nasal mask. The inspiratory conduit 106 can also connect to other types of patient interface, such as a full face (oronasal) mask, a total face mask configured to cover an entire patient face, an endotracheal tube, nasal pillows, or others. The inspiratory conduit 106 can also be connected to any suitable patient interface. The system can optionally include an expiratory conduit. The expiratory conduit can connect the user and the gases source (for example, the ventilator) to return the expired gases to the gases source.

A heating element 110 (such as one or more heater wires) can be provided within the inspiratory conduit 106. The heating element 110 can help prevent condensation of the humidified gases within the inspiratory conduit 106. The heating element 110 can also optionally be in electrical communication with the controller in the heater base unit 102. As shown in Figures 1A and 1C, an inspiratory conduit heating element adaptor cable 128 can have two connectors at two ends of the cable 128 for electrically coupling the heating element 110 to the heater base unit 102 (such as to the controller of the heater base unit 102). The heating element adaptor cable 128 can facilitate an easy connection between the heating element 110 and the heater base unit 102. Alternatively, a cable can extend from the heater base unit 102 to electrically couple a heating element to the heater base unit 102. As another alternative, the inspiratory conduit can include electrical contacts to couple a heating element to the heater base unit 102. The heating element 110 can be controlled by the controller in the heater base unit 102, including the controlling of power to the heating element 110 by the controller. The heating element 110 in the inspiratory conduit 106 may reduce condensation and may also ensure that the temperature and/or humidity of gases are maintained within a predetermined range. The heating element adaptor cable 128 can also include an ambient temperature sensor 126, which can allow the system 100 to adjust the heating element 110 power and/or the heater plate heating element power to be based at least in part on ambient temperatures (as will be described in greater detail below). The ambient temperature sensor can alternatively be located anywhere that is exposed to the ambient air. Ambient air can refer to ambient air around the respiratory humidifier system.

As shown in Figure 1C, a heating element indicator 130 can be embedded into the connector that couples to the heater base unit 102. Alternatively, the heating element indicator 130 can be located on the heater base unit 102. The heating element indicator 130 can be illuminated when a properly functioning heating element 110 of the inspiratory conduit 106 is connected to the heater base unit 102. When the heating element indicator 130 is illuminated, the system 100 can heat the gases inside the inspiratory conduit 106 via the heating element 110 to minimize condensate, in addition to heating the gases passing through the humidifier chamber 103 via the heater plate 120. If the heating element 110 is malfunctioning or not connected, the system 100 can heat the gases only by heating the water in the humidifier chamber 103 via the heater plate 120. Alternatively, the heating element indicator 130 can be illuminated when there is a fault or a disconnection of the adaptor cable 128 from the heating element 110. The illuminated indicator 130 can act as a visual message or a visual warning. The indicator 130 may not be illuminated if the heating element 110 is functioning correctly.

The controller of the respiratory humidifier system 100 can control at least the heater plate 120, and preferably or optionally also the heating element 110, without additional sensors (for example, in the humidifier chamber, at the chamber inlet, at the chamber outlet, in the inspiratory conduit, and/or elsewhere in the system) other than the heater plate temperature sensor(s) and the ambient temperature sensor. This can be achieved by estimating the flow rate of gases through the respiratory humidifier system 100 using parameters already available to the controller. For a given respiratory humidifier system, the controller can determine an appropriate level of power to energize the heater plate 120 to generate humidity and heat the gases. The energization of the heater plate can be controlled to generate a predetermined amount of humidity. Additionally, the parameters can also optionally be used by the controller to provide a more appropriate level of power to the heating element 110.

As shown in Figure 1C, a front panel of the heater base unit 102 can include a plurality of user controls and indicators, such as a power button 132, a humidity setting push button 134, and a plurality of (such as three, four, five or more) humidity setting indicators 136 (which can include LED lights) next to the humidity setting push button 134. The locations, shapes, and sizes of the user controls and indicators are not limiting. There can be, for example, four levels of humidity settings available which are indicated by the four humidity setting indicators 136. The four humidity settings can correspond to different types of therapies provided to a user. For example, the highest amount of humidity can be selected when the humidifier is operating in an invasive therapy mode. The amount of humidity can be selected based on therapeutic requirements or therapy type or can be predefined. Alternatively, the humidifier system 100 can include a controller that is configured to automatically select the amount of humidity to be delivered based on a therapy mode, the user, or the type of therapy being applied to the user. Optionally, the humidifier system 100 can include a display or a touch screen that may communicate information to the user. The touch screen can also be configured to receive inputs from the user.

The humidity level can be adjusted by pressing the humidity settings push button 134, which can also be a momentary push button. The front panel can also include a plurality of alarm indicators 138 (which can include LED lights) to indicate the following nonlimiting examples of conditions: "water out" condition (including low water and water-out), heating element adaptor not connected, audible alarm muted, and a "See Manual" indication used to indicate that a fault has occurred within the system 100.

The system 100 can be suitable for providing respiratory therapy for different purposes, such as for critical care (for example, in the hospital) and home care. The system 100 can be suitable for providing invasive, non-invasive and high flow therapies for adult and/or pediatric users.

In a humidifier system such as the example systems described herein, excessive energy transfer to a patient is undesirable for safety purposes. Such energy transfer is part of the enthalpy of the humidifier system. Key variables that influence enthalpy in a humidifier system are: flow rate, water temperature, and ambient temperature, among others. In such a humidifier system, energy transfer between the humidifier and the breathing gases primarily occurs via the evaporation process. The energy transfer rate can be determined based on the evaporation rate. The evaporation rate is determined by the water temperature and flow rate. The flow rate dictates the volume of gas exiting the system per unit of time. Throughout this disclosure any method, system, or apparatus used to reduce, control, or affect enthalpy can be understood to also reduce, control or affect specific enthalpy. Specific enthalpy can be defined as the total heat content contained in a substance per its volume. A chamber outlet enthalpy can be determined (for example calculated) using the total energy transfer at a given water temperature over a unit of time, divided by the total flow exiting the system over the same unit of time. That is, the chamber outlet enthalpy is positively correlated with the water temperature and inversely correlated with the flow rate (that is, higher enthalpy being more of a concern at low flow rates as described above). The chamber outlet enthalpy can also be empirically derived for a given flow rate or a given water temperature, such as for the worst case enthalpy conditions of the humidifier system.

The power to the heating element of the inspiratory conduit and the ambient temperature can affect how much of the enthalpy in the humidifier system reaches the user end. The combination of these parameters needs to be maintained within safe operating conditions for the patient. A higher power to the heating element of the inspiratory conduit can result in a higher enthalpy of the gases at the user end. A higher ambient temperature means less energy dissipation can occur via the wall of the inspiratory conduit. Given the known chamber outlet enthalpy (as estimated above) and a known limit on user end enthalpy and/or temperature, it can be determined how much energy must be dissipated by the inspiratory circuit for the enthalpy of the gases at the user end to remain at a safe level. The determined amount of energy dissipation can be translated into a maximum allowable power to the heating element of the inspiratory conduit for a given ambient temperature.

For humidifier systems that can deliver higher humidity at higher flow rates, the maximum heater plate and/or water temperature can be higher. Therefore, the chamber outlet enthalpy can also be higher. Additional protection may be required to keep the user end enthalpy of the gases within a safe limit for the user.

As will be described in detail below, the controller of the respiratory humidifier system 100 can determine and/or apply a power limit on the heating element 110 using inputs such as from the heater plate temperature sensor, and/or heater plate power, and/or the ambient temperature sensor, and/or other parameters of the heater plate. In some configurations where it is difficult to directly cool the heater plate and/or rapidly reduce the water temperature, the system 100 can control the power to the heating element 110, for example, by keeping the power under a limit or turning it off, to dissipate the higher level of enthalpy. The controller may not necessarily need inputs from additional sensors for that determination, such as additional sensors in the gas flow path, including but not limited to a flow sensor, a pressure sensor, a humidity sensor, a chamber inlet temperature sensor, a chamber outlet temperature sensor, and/or a patient end temperature sensor. In some configurations, the controller does not receive inputs from any additional sensors in the gas flow path including the following types of sensors: flow sensors, pressure sensors, humidity sensors, chamber inlet temperature sensors, chamber outlet temperature sensors, and/or patient end temperature sensors. In some configurations, the controller does not receive inputs from any additional sensors in the inspiratory conduit. In some configurations, the controller does not receive inputs from any sensors downstream of the humidifier chamber. In some configurations, the controller only has access to sensors in the humidifier chamber and/or sensors for ambient conditions. In some configurations, the controller only receives inputs from sensors for heater plate temperature, heater plate power, heating element power, and ambient temperature. In some configurations, the controller only receives inputs from sensors for heater plate temperature, heater plate power, and heating element power. In some configurations, the controller only receives inputs from sensors for heater plate temperature, heater plate power, and ambient temperature. The controller may not receive inputs from additional sensors as described above, because those additional sensors are not included in the system or have not been properly connected to the controller during setup. The determination and/or application of the power limit can be performed using software that may limit the maximum power delivered to the heating element 110. Additionally and/or alternatively the power limit can be determined and/or applied using hardware components which may be configured to disable power to the heating element 110 and/or heater plate heating element. In some configurations, hardware components are discrete physical components. In some configurations, hardware components are analog circuit components. In some configurations, hardware components are FPGAs (field programmable gate arrays). In some configurations, application of the power limit can include triggering a protection circuit.

In this disclosure, the terms controller and hardware controller can be interchangeable. For example, a controller or hardware controller can be a microprocessor or a CPU with software instructions to control other components. In another configuration, a controller or hardware controller can be an analog circuit or hardware circuit component that is able to control other components by shutting off the ability for a component of the respiratory humidifying system from receiving power. In this way, the controller or hardware controller controls a component by providing or not providing power to the component.

As described herein, the system 100 may not have any sensors in the gases flow path. This means that there may not be direct feedback or information of the enthalpy of the gases. Optionally, the system 100 and each of the other systems described herein can include a sensor at the chamber inlet, chamber outlet and/or patient end. The sensor can be, for example, a temperature sensor.

Under some situations an undesirable, higher than safe level of humidity and/or enthalpy may be delivered due to system characteristics. For example, a large flow drop (for example, from about 30-40 L/min to about 5 L/min) would cause increased enthalpy at the user end that is undesirable as the high water temperature necessary for gases (for example to produce sufficient humidity) at a higher gas flow rate is temporarily applied to gases at a lower gas flow rate. In some cases, this may lead to the gases having too high enthalpy. It is possible for enthalpy at the user end to remain below a certain limit by limiting the maximum heater plate and/or water temperature that can be applied, but this limitation restricts the humidity performance under a higher flow rate condition. In order to accommodate greater flow rate ranges and humidity levels, the humidifier system can apply an enthalpy control method such as disclosed herein.

In some situations, for example situations with the above conditions, multiple enthalpy protections may be required (for example, by both limiting heater plate power for example, by software in a controller), or by turning off the heater plate and/or heater wire (for example, a hardware component to turn off the heater plate and/or heater wire). Hardware components for over enthalpy protection can be used for protecting against fault conditions or problems with software. Software can be used to protect against different scenarios during operation.

The patient end enthalpy limit can be expressed as a specific enthalpy of from about 122 kJ/m$^3$ to 216 kJ/m$^3$, or from 150 kJ/m$^3$ to 216 kJ/m$^3$, or from 185 kJ/m$^3$ to 210 kJ/m$^3$, or from 195 kJ/m$^3$ to 205 kJ/m$^3$. Alternatively, the patient end enthalpy limit can be expressed as a temperature of the gases that are substantially fully saturated with water vapor of from 32°C to 45°C, or from 35°C to 45°C, or from 38°C to 42°C.

Another patient end enthalpy limit can be expressed as a specific enthalpy of from 195 kJ/m$^3$ to 441 kJ/m$^3$, or from 195 kJ/m$^3$ to 400 kJ/m$^3$, or from 195 to 350 kJ/m$^3$, or from 195 kJ/m$^3$ to 250 kJ/m$^3$, or from 195 kJ/m$^3$ to 205 kJ/m$^3$. Alternatively, the patient end enthalpy limit can be expressed as a temperature of the gases that are substantially fully saturated with water vapor of from 43°C to 63°C, or from 43°C to 50°C, or from 43°C to 45°C. The generally lower limit described in the preceding paragraph can be used to prevent over-enthalpy delivery. The generally higher limit described in this paragraph can prevent more significant over-enthalpy delivery. The generally higher limit can be used as redundancy over the generally lower limit, and/or can act as a back-up to the generally lower limit.

Over-enthalpy protection can also be incorporated in other types of respiratory and/or surgical humidifier system disclosed herein, which can include other sensor(s) and/or have different configurations. These humidifier systems can include at least one heater plate temperature sensor and/or at least one ambient temperature sensor. Non-limiting example humidifier systems that can benefit from over-enthalpy protection are described with reference to Figures 2A-2E.

As shown in Figures 2A and 2B, a respiratory humidifier system 200 can include a blower unit 203 and a humidifier 201. The blower unit 203 can have an internal compressor unit, flow generator or fan unit 213. Gases from the atmosphere can enter a housing of the blower unit 203 via an atmospheric inlet 240, and can be drawn through the fan unit 213. The output of the fan unit 213 can be adjustable so that the fan speed is variable. The pressurized gases stream can exit the fan unit 213 and the blower unit 203 and can travel via a connection conduit 204 to a humidifier chamber 205 of the humidifier 201. The pressurized gases stream can enter the humidifier chamber 205 via an inlet port 223.

The blower unit can also optionally be replaced by a ventilator having fans or turbines configured to generate a gases flow. The ventilator can receive gases from a compressed gases source, such as a tank. The ventilators can also use one or more valves to control the delivery of gases to the humidifier chamber 205.

The respiratory humidifier system 200 can also provide oxygen ($O_2$) or an $O_2$ fraction to the user. The system 200 can receive $O_2$ from the remote source and/or by blending atmospheric air with incoming $O_2$ from the remote source. The blending of atmospheric air and incoming $O_2$ can occur via a Venturi or a similar inlet located in the control unit 203.

Figure 2C illustrates an example respiratory humidifier system 200 that has an integrated blower/heater base unit 210. The system in Figure 2C operates in a similar manner to the respiratory humidifier system 200 shown in Figure 2A and 2B, except that the heater base unit has been integrated with the blower to form an integrated unit 210 with a housing 203.

Similar to the humidifier chamber 103 described above, the humidifier chamber 205 in Figures 2A-2C can contain a volume of water 220. When in use, the humidifier chamber 205 can be engaged with a heater base unit 221 of the humidifier 201 or the integrated unit 210 (for example, by being placed in contact with and/or on top of a heater plate 212). The heater plate 212 can be energized to heat a conductive base of the humidifier chamber 205 and thereby heat the content (such as the volume of water 220) in the humidifier chamber 205. The gases stream entering the humidifier chamber 205 via inlet port 223 becomes heated and humidified and can exit the humidifier chamber 205 via an outlet port 209 to enter an inspiratory conduit 206.

The heated, humidified gases can travel along the length of the inspiratory conduit 206 and be provided to the user 202 via a patient interface 207. The inspiratory conduit 206 in Figures 2A-2C can also optionally be heated via a heating element (such as a heater wire 210) to help prevent condensation of the heated, humidified gases. The patient interface 207 shown in Figures 2A and 2C is a nasal mask, which surrounds and covers the nose of the user 202. However, a nasal cannula (as shown in Figure 2B), full face mask, endotracheal tube, tracheostomy fitting, or any other suitable patient interface could be substituted for the nasal mask shown.

A central controller or control system can be located in the blower unit 203 (hardware controller 208a), the heater base unit 221 (hardware controller 208b), or both (for example, having separate blower controller 208a and humidifier controller 208b being in electrical communication with each other via connecting cables or others, or a central controller 208 as shown in Figure 2C). The blower controller 208a and the humidifier controller 208b can optionally be in a master-servant relationship (for example, one of the controllers can control the functions of the other controller) or in a peer relationship (for example, each controller can function independently of the other). For instance, the humidifier controller 208b can be an independent unit configured for use with any type of gases source.

The control system can receive user inputs via user controls 211 located on the heater base unit 221, the blower unit 202, or both. The control system can also receive inputs from sensors located at various points throughout the system 200. Similar to the respiratory humidifier system 100 described above, the respiratory humidifier system 200 can include a heater plate temperature sensor 262 located adjacent to or at (for example, immediately below) a top plate of the heater plate 212. The heater plate temperature sensor 262 can be configured to measure a temperature of the heater plate 212.

As shown in Figures 2A-2C, the respiratory humidifier system 200 can include additional temperature sensors. An ambient temperature sensor 260 can be included in the humidifier system 200 of Figures 2A-2C. The ambient temperature sensor 260 can be located anywhere where the ambient temperature sensor 260 is exposed to the ambient environment. The ambient temperature sensor 260 can be configured to measure the temperature of the incoming air from the atmosphere. An outlet temperature sensor can optionally be located at or near a humidifier chamber outlet 209, or at a chamber end (opposite the user end) of the inspiratory conduit 206. The outlet temperature sensor can be configured to measure a temperature of the gases stream exiting the humidifier chamber 205. A user end temperature sensor 215 can optionally be located at the user end of the inspiratory conduit 206. The user end temperature sensor 215 can also optionally be located in or on the patient interface 207.

The respiratory humidifier system 200 can optionally include a flow sensor configured to measure the gases flow through the system 200. The flow sensor can be located upstream of the fan unit 213, downstream of the fan unit 213, or at other locations. For example, the flow sensor 263 can be located at or near the humidifier chamber outlet 209, the chamber end of the inspiratory conduit 206, and/or adjacent to the outlet temperature sensor at or near the humidifier chamber outlet. The sensor 263 can optionally include both temperature and flow sensors. The controllers 208a, 208b can also optionally include one or more other sensors 250, 280, 290, which can measure the humidity, temperature, pressure, flow, and/or other characteristics of the gases flow.

Alternate configurations of Figures 2A, 2B, and 2C are shown in Figures 2A2, 2B2, and 2C2, respectively. In the configurations represented in Figures 2A2, 2B2, and 2C2, there are no user end temperature sensor 215 or flow sensor 263 as compared to Figures 2A, 2B, and 2C. In some configurations, there are no sensors downstream of the humidifier chamber 205. In some configurations, there is no outlet temperature sensor at outlet 209.

As shown in Figures 2A2-2C2, the respiratory humidifier system 200 can include additional temperature sensors. An ambient temperature sensor 260 can be included in the humidifier system 200 of Figures 2A2-2C2. The ambient temperature sensor 260 can be located anywhere where the ambient temperature sensor 260 is exposed to the ambient environment. The ambient temperature sensor 260 can be configured to measure the temperature of the air in the atmosphere that is external to the gases flow path. The adaptor cable 128 of Figure 1C can also include an ambient temperature sensor 126.

In response to the user input from controls 211 and/or the input signals received from the sensors, the control system can determine one or more control outputs, which can send signals to adjust the energization of the heater plate 212, the speed of the fan unit 213, the power to the heating element of the inspiratory conduit 206, and/or others.

In any of the respiratory humidifier systems 100, 200 described above, a temperature probe can also optionally be placed within the volume of water in the humidifier chamber. Additionally and/or alternatively, contactless temperature sensors (such as infrared sensors) can also be used to measure a temperature of the heater plate and/or the content of the humidifier chamber, and/or a temperature of the gases path.

The over-enthalpy protection methods and/or circuits described below can be based on readings from a heater plate temperature sensor and an ambient temperature sensor without requiring any gases path sensors. Accordingly, such over-enthalpy protection methods and/or circuits can be implemented in respiratory humidification systems that do not have sensors in the gases flow path. Alternatively, readings from the temperature probe, the contactless temperature sensor, and/or any other temperature sensor located downstream of the gases inlet of the humidifier chamber and/or close to the heater plate can also optionally be used as the heater plate temperature input in the over-enthalpy protection methods described below.

The systems in Figures 2A-2C can also optionally include an expiratory conduit. The expiratory conduit can connect the user and the gases source (for example, the ventilator) to return the expired gases to the gases source.

A schematic representation of a high flow therapy system 10 is provided in Figure 2D. The system 10 can include a main device housing 100 that houses an integrated blower and heater base unit. The main device housing 100 can contain a flow generator 11 that can be in the form of a motor/impeller arrangement, a humidifier or humidification chamber 12, a controller 13, and an interface 14. The interface 14 can include a display and input device(s) such as button(s), a touch screen, a combination of a touch screen and button(s), or the like. The controller 13 can include one or more processors and can be configured or programmed to control the components of the apparatus, including but not limited to operating the flow generator 11 to create a flow of gases for delivery to a user, operating the humidifier 12 (including a heater plate configured to be energized to heat water contained in a humidifier chamber in contact with the heater plate) to humidify and/or heat the gases flow, receiving user input from the interface 14 for reconfiguration and/or user-defined operation of the respiratory system 10, and outputting information (for example on the display) to the user. Alternatively, each of the flow generator 11 or the humidifier 12 can be controlled by an independent controller.

With continued reference to Figure 2D, an inspiratory conduit 16 can be coupled to a gases flow outlet 21 in the main device housing 100 of the respiratory system 10, and be coupled to a patient interface 17, such as a non-sealing interface like a nasal cannula with a manifold 19 and nasal prongs 18. The inspiratory conduit 16 can also be coupled to a face mask, a nasal mask, a nasal pillow mask, an endotracheal tube, a tracheostomy interface, or others. The main device housing 100 of the respiratory system 10 can also include I/O 14 for user controls and physically connecting to multiple other devices and/or systems (for example, connections such as a wired connections, USB ports, HDMI ports, and the like). The main device housing 100 of the respiratory system 10 can also include a chipset for wireless communication 15 (for example, Bluetooth, WiFi, 4G/5G, NFC, and other wireless communication signals and systems).

The gases flow can be generated by the flow generator 11, and be humidified, before being delivered to the user via the inspiratory conduit 16 through the patient interface 17. The controller 13 can control the flow generator 11 to generate a gases flow of a desired flow rate, and/or one or more valves to control mixing of air and oxygen or other breathable gas. The controller 13 can control a heating element in the humidifier chamber 12 to heat the gases to a desired temperature that achieves a desired level of temperature and/or humidity for delivery to the user. The inspiratory conduit 16 can have a heating element 16a, for example, a heater wire, to heat gases flow passing through to the user. The heating element 16a can also be under the control of the controller 13.

High flow therapy can be effective in meeting or exceeding the user's inspiratory demand, increasing oxygenation of the user and/or reducing the work of breathing. Additionally, high flow therapy may generate a flushing effect in the nasopharynx such that the anatomical dead space of the upper airways is flushed by the high incoming gases flow. The flushing effect can create a reservoir of fresh gases available for each and every breath, while minimizing re-breathing of carbon dioxide, nitrogen, etc. For high flow therapy, a blower or alternatively a wall source with a flow and/or pressure regulator can supply the gases.

The patient interface for use in a high flow therapy can be a non-sealing interface to prevent barotrauma, which can include tissue damage to the lungs or other organs of the user's respiratory system due to difference in pressure relative to the atmosphere. The patient interface can be a nasal cannula with a manifold and nasal prongs, and/or a face mask, and/or a nasal pillows mask, and/or a nasal mask, and/or a tracheostomy interface, or any other suitable type of patient interface.

Figure 2E illustrates schematically using an example surgical system 1 during an example medical procedure. The example medical procedure shown in Figure 2E is a laparoscopic procedure, but can be any other medical procedure such as open surgery, for example. The surgical system humidifies a surgical cavity (for example, the pneumoperitoneum as shown in Figure 2E). The humidifier 201 can humidify the tissue in the surgical cavity and prevent damage to the tissue due to drying, including desiccation or low temperature. As shown in Figure 2E, the patient 2 can have a cannula 207 inserted within a cavity of the patient 2 (for example, an abdomen of the patient 2 in the case of a laparoscopic surgery).

As shown in Figure 2E, the cannula 207 can be connected to a gases delivery conduit 206 (for example, via a Luer lock connector 4). The cannula 207 can be used to deliver gases into a surgical site, such as within the cavity of the patient 2. The cannula 207 can include one or more passages to introduce gases and/or one or more surgical instruments into the surgical cavity. The surgical instrument can be a scope, electrocautery tool, or any other instrument. The surgical instrument can be coupled to an imaging device, which can have a screen. The imaging device can be part of a surgical stack, which can include a plurality of surgical tools and/or apparatuses.

The surgical system can also optionally include a venting cannula, which can have substantially the same features as the cannula 207. The venting cannula may include a valve that allows venting. The valve can be automatically controlled by a controller associated with the gases source (for example, insufflator) or by a controller in the humidifier. The valve can also be manually actuated (for example, by turning a tap by hand or by a foot pedal, or otherwise). The venting cannula can be coupled to a filtration system to filter out smoke and the like. The venting cannula can also alternatively be coupled to a recirculation system that is configured to recirculate the gases from the surgical cavity back to the insufflator for re-delivery into the surgical cavity. The gases can be filtered and/or dehumidified prior to being returned to the insufflator. In certain configurations, the cannula may include two or more passages. One passage can be configured to deliver gases and/or the medical instrument into the surgical cavity. Another passage can be configured to vent gases out of the surgical cavity. The venting passage may include a valve and/or passive vent openings. The cannula 207 may also include a retaining arrangement (such as ribs and/or the like) to retain the medical instrument (such as a scope) in a substantially concentric orientation relative to the delivery passage.

The gases delivery conduit 206 can be made of a flexible plastic and can be connected to a humidifier chamber 205 of the humidifier 201 at an inlet 223. The humidifier chamber 205 can optionally or preferably be in serial connection to a gases supply 9 via a further conduit 204. The gases supply or gases source can be an insufflator, bottled gases, or a wall gases source. The gases supply 9 can provide the gases without humidification and/or heating. A filter 6 be connected downstream of the humidifier 201's outlet 209. The filter can also be located along the further conduit 209, or at an inlet of the cannula 205. The filter can be configured to filter out pathogens and particulate matter in order to reduce infection or contamination of the surgical site from the humidifier or gases source. The gases supply can provide a continuous or intermittent flow of gases. The further conduit 204 can also preferably be made of flexible plastic tubing.

The gases supply 9 can provide one or more insufflation gases, such as carbon dioxide, to the humidifier chamber 205. The gases supply can provide a continuous gases flow or an intermittent gases flow. The gases can be humidified as they are passed through the humidifier chamber 205, which can contain a volume of water 220. In some configurations, the gases supply can also be directly connected to the cannula 205 without a humidifier unit.

The humidifier of the surgical system 1 in Figure 2E can optionally incorporate any of features of the humidifier 201, including the humidifier unit 221 and humidification chamber 205 shown in Figures 2A-2D. Alternatively, the humidifier can be configured to deliver humidified gases during any other suitable medical procedure, such as open surgery, for example.

**In** some configurations, a respiratory humidifier system may have limited sensors to detect conditions indicative of unsafe enthalpy. For example, the respiratory humidifier system may not have any sensors at the patient end of the inspiratory conduit and cannot determine a flow rate of the gases, temperature of the gases, and the like at the patient end of the inspiratory conduit. In some configurations, a respiratory humidifier system may not have sensors downstream from the heater plate to detect conditions indicative of unsafe enthalpy at the patient.

**In** such example configurations, one or more parameters of the heater plate (for example, heater plate temperature (HPTemp), heater plate power (HPPower)), and ambient temperature can be used by a controller to adjust heating element power (for example, the heating element or heater wire in the inspiratory conduit) to maintain user end enthalpy below an unsafe limit. In some configurations, a power limit can be used to limit the amount of power able to be delivered to the heating element. In some configurations, controlling the power to the heating element includes controlling the power to, or below, a power limit.

Other forms of controlling the power to, or below, a power limit may include directly controlling power in the heating element to, or below, a power limit. In some configurations, disabling a component or the entire system if the power limit is exceeded is a way to control the power to below a power limit. For example, the heater plate and/or the heating element can be disabled if the power limit to the heater plate and/or heating element is exceeded.

Additional factors can be used by the controller in adjusting power to the heating element. These factors can include a flow indicator (Kappa) and estimated humidity. In some configurations the flow indicator (Kappa) and estimated humidity can be determined as a function of HPTemp, HPPower and ambient temperature.

In some configurations, the respiratory system can have two or more conduits which each have one or more conduit heating elements. All forms of enthalpy protection, power limiting, or power disabling, and the like described in this disclosure can be applied to each conduit and/or to one or more conduit heating elements, individually or in combination.

Certain methods of power control may be described as a software or hardware method of power control. However, each method of power control can be implemented in software of the controller or in a hardware component. Having both software and hardware methods of power control can provide and/or increase single fault tolerance of the over enthalpy protections in the respiratory humidifier system.

Having no sensors in the inspiratory conduit or downstream from the heater plate (or any other combination) reduces the complexity of the respiratory humidifier system. Similarly, using fewer sensors or limiting the types of sensors can also reduce complexity. This can result in cost savings in manufacturing and cost savings for customers. Furthermore, the respiratory humidifier system may be easier and quicker to deploy. Similarly, training operators of the respiratory humidifier system may be easier and quicker if the system is less complex with fewer sensors and fewer types of sensors. Additionally, a less complex respiratory humidifier system has fewer components and subsystems which can become faulty, broken, or corrupted thus reducing costs of maintenance by reducing the troubleshooting efforts, likelihood of breakdown, and ease of maintenance.

A user of the humidifier systems disclosed herein can be at a risk of over-enthalpy such as when the gases experience a large drop from a high flow rate to a low flow rate as described above, which may result in the heater plate temperature being excessively high (for example, above 50°C to 90°C, or above 60°C).

Figure 3 shows the undesirable effects during a large drop of the flow rate introduced at around 2 hours. In the example shown in Figure 3, the flow rate drops from about 30 L/min to about 5 L/min. The heater plate power (HPPower) and the heater plate temperature (HPTemp) drop in response to the large drop in the flow rate. The power (for example, the steady state power) to the heating element of the inspiratory conduit (SSHWDuty), on the other hand, remains substantially constant as the SSHWDuty is not directly related to or a function of the HPPower or HPTemp. SSHWDuty is the power (for example, in the form of a duty cycle) provided to the heating element of the inspiratory conduit to maintain temperature and reduce condensation when delivering the target or desired therapy level for given system conditions (for example, ambient temperature under steady-state conditions) and for a given conduit type (for example, due to the heater wire characteristics such as heater wire resistance). The maximum power limit to the heating element of the inspiratory conduit (MaxHWDuty) represents a power limit that corresponds to a maximum allowable patient end temperature. Exceeding this maximum allowable patient end temperature (and/or enthalpy) can cause significant energy being delivered to the patient. As shown in Figure 3, the SSHWDuty (without any attempt to limit the power) exceeds the MaxHWDuty during the flow drop event for a period of time, which is illustrated by a boundary 340.

Anywhere this disclosure refers to duty cycle (whether in the name of a variable, label or description with regards to the written disclosure herein, the disclosure's graphs, or the disclosure's figures), duty cycle is just one example of how power can be adjusted or controlled with relation to the heater plate or the heating element. For example, SSHWDuty can refer to the steady state heating element duty cycle, but it can also refer to any type of heating element power (including but not limited steady state heating element power or steady state heating element duty cycle), where the heating element refers to the heating element of the inspiratory conduit. Power can be adjusted by adjusting one or more parameters of the heating element for example a voltage or current duty cycle of heating element, the current through the heating element or the voltage on the heating element. It is to be understood that anyway in which power can be adjusted can be used throughout this disclosure. It is to be understood that power to the heater plate can also be adjusted in more ways than just by adjusting duty cycle. Power to the heater plate can be adjusted by adjusting one or more parameters of the heater plater for example a voltage or current duty cycle of heater plater, the current through the heater plater or the voltage on the heater plater.

The respiratory humidifier system can control or limit power via any power control or power limit methods. For example, power can be controlled or limited by controlling a heater wire (or heating element) voltage, or a heater wire current and/or heater wire resistance. For example, the system can calculate a power limit in Watts and then calculate a corresponding duty cycle limit to reach that power limit. The duty cycle may additionally be based on a characteristic of the mains voltage supply (for example frequency and/or voltage).

SSHWDuty can refer broadly to the power which the controller of the heating element of the inspiratory conduit determines to provide to the heating element of the inspiratory conduit, independent of any power limits. At steady state the SSHWDuty may be a steady state power (for example as a steady state duty cycle SSHWDuty). SSHWDuty is determined by a controller of the system based on ambient temperature and conduit characteristics (for example, the conduit could have different characteristics concerning heating element resistance and the like).

MaxHWDuty refers to the maximum power limit to the heating element that corresponds to a maximum allowable temperature (and/or enthalpy) for the patient end of the inspiratory conduit. MaxHWDuty in the context of the specification refers to a theoretically (for example calculated) or empirically derived maximum inspiratory conduit power (for example as described in more detail below). One goal of the system is to ensure that the power delivered to the inspiratory conduit power is less than the maximum inspiratory conduit power (MaxHWDuty). Other examples and variables are disclosed above and below in this disclosure.

The MaxHWDuty can be empirically derived using a patient end sensor, such as a patient end temperature sensor, and a closed loop control algorithm. Alternatively, in a respiratory humidifier system that does not include a patient end temperature sensor or other sensors in the gases flow path, the controller of such a system can derive the MaxHWDuty using other available parameters. A respiratory humidifier system without a patient end temperature sensor or other sensors in the gases flow path can have decreased cost due to fewer components, increased reliability by having less failure points at different components, and better usability since fewer sensors need to be properly setup by the system user. Such a system could also have reduced complexity for manufacturing/assembly with fewer components that need to be added to the system. Such a system could also have reduced complexity for ongoing operation by a user by reducing the need for routine maintenance and testing for accuracy and precision of sensors. In a respiratory humidifier system that does not include a temperature sensor in the gases flow path, a MaxHWDuty value can be estimated using the methods described herein. Similarly, the methods described herein can be used as a backup or fail-safe to prevent patient harm even if the respiratory humidifier system includes sensors in the gases flow path. The methods described herein can also be used to determine if sensors in the gases flow path are compromised or unreliable.

The respiratory humidifier system can prevent significant enthalpy from being delivered by not allowing the heater wire power (SSHWDuty) to exceed the maximum power limit to the heating element that corresponds to a maximum allowable temperature and/or enthalpy for the patient end of the inspiratory conduit (MaxHWDuty) during any event, such as during the flow drop event described above. The controller of the system can output a power to the heating element of the inspiratory conduit that can be up to, but not exceeding, a maximum allowable power. Additionally, keeping the SSHWDuty to below or approximate the MaxHWDuty during an over-enthalpy event can reduce the amount of condensation formed in the inspiratory conduit. Two ways of ensuring that the SSHWDuty does not exceed the MaxHWDuty can include creating a power offset that is applied only during a flow drop event (Method 1) or determining a limit to the MaxHWDuty that is applied across all conditions of the respiratory humidifier system (Method 2). Both Method 1 and Method 2 can be implemented separately or together, and both Method 1 and Method 2 can be implemented in the software of a controller or implemented in a hardware component such as an analog circuit or FPGA.

The systems disclosed herein can implement processes to prevent over-enthalpy (for example, using a hardware controller disclosed herein). Example parameters can include inputs of a heater plate temperature, a heater plate power, and an ambient temperature, which can be received by the hardware controller, for example, from the sensors described herein. Additionally and/or alternatively, preventing over-enthalpy can be achieved using hardware components (for example protection circuits) implementing the process(es) as disclosed herein. The over-enthalpy protection can be important in all humidification systems, including respiratory and surgical humidification systems. The over-enthalpy protection can be implemented without necessarily requiring inputs from sensors in the gases flow path of the humidification system.

Method 1 decreases power to the heating element of the inspiratory conduit during a flow drop event such that the power to the heating element is below a MaxHWDuty using parameters available to the respiratory humidifier system as noted above. Rather than directly calculating the MaxHWDuty based on readings from sensors in the gases flow path (because the system does not have sensors in the gases flow path, for example, from a patient end temperature sensor) a power offset is directly applied to, and is subtracted from, the SSHWDuty that is being calculated (for example the SSHWDuty which is being calculated by the controller for the heating element of the inspiratory conduit). The power offset can be designed in such a way that it only effects the power delivered to the heating element of the inspiratory conduit (SSHWDuty) during the flow drop event (or other event where excess enthalpy may be provided to the patient). Outside of the flow drop event, the power offset may have no effect and SSHWDuty is applied.

The power offset can be calculated using the HPPower, HPTemp and/or the ambient temperature readings. The power offset calculation involves at least two terms. The power offset may be based on Kappa and HPTemp. In some embodiments, the generalized function for the power offset calculation is Kappa * HPTemp. The first term, Kappa can be a flow indicator. That is, Kappa can be a parameter indicative of a flow rate of the flow of gases. The parameter indicative of a flow rate of the flow of gases can be based on a thermal conductivity value, or a value indicative of a thermal conductivity, between the heater plate and the flow of gases. The value indicative of a thermal conductivity value can be based on the HPPower, HPTemp, and/or the ambient temperature reading. The second term can be the HPTemp. These terms can dictate the magnitude of the power offset and HPTemp can be primarily responsible for the power offset's slow decay back to normal therapy delivery as the temperature of the water in the chamber cools. The power offset does not need to be applied under a threshold HPTemp value regardless of the value of Kappa as enthalpy is inherently below the significant level when the HPTemp is under that certain HPTemp value. The power offset does not need to be applied above a threshold Kappa value regardless of the value of HPTemp as enthalpy may be inherently below the significant level when Kappa is above that threshold Kappa value. Therefore, the power offset calculation may calculate a power offset, but the power offset may not be applied if the HPTemp is below a threshold and Kappa is above a threshold.

Figure 4A illustrates Kappa (K), a value indicative of thermal conductivity and thus also indicative of a flow rate, which is plotted against the right y-axis, and can be calculated using the equation:

$$thermal\ conductivity, \kappa = \frac{HPPower}{HP\ Temp - ambient\ temperature}.$$

In Figure 4A, the other terms are plotted against the left y-axis. As shown in Figure 4A (and Figure 3 as described above), when a large flow drop occurs at approximately the 2 hour time point (2.0 Hrs), the HPPower drops followed by a corresponding drop in HPTemp. The overall effect of this is that Kappa decreases. OffsetHWDuty is the power to the inspiratory conduit heating element (for example, the heater wire) based on a power offset is applied to SSHWDuty (for example, a target heater wire power.) OffsetHWDuty, is calculated as: OffsetHWDuty = SSHWDuty - the power offset. Although not plotted directly in Figure 4A, it can be seen that the power offset is the positive difference between the SSHWDuty and the OffsetHWDuty. In other words, the power offset becomes positive when the OffsetHWDuty drops below the SSHWDuty.

The power offset becomes positive, that is, the OffsetHWDuty drops below the SSHWDuty, only when both Kappa drops below a reference flow indicator threshold (for example a Kappa threshold) and the HPTemp exceeds a heater plate temperature threshold. In all other cases, the power offset is set to zero. Preferably, operation of the respiratory humidifier system results in an OffsetHWDuty that is as close to SSHWDuty as possible without exceeding the MaxHWDuty. In some configurations, OffsetHWDuty can be within a tolerance threshold of the MaxHWDuty when the power offset is positive. The heater plate temperature threshold can be within the range of about 55°C to about 70°C, or about 55°C to about 60°C, or about 60°C to about 65°C, or about 65°C to about 70°C.

An example process according to Method 1 to determine and/or apply a power offset on the heating element of the inspiratory conduit under an over-enthalpy scenario is illustrated in Figure 3A. As shown, the hardware controller of the system can receive a heater plate temperature value at step 300, an ambient temperature value at step 302, and a heater plate power value at step 304. The hardware controller can calculate a flow indicator of the flow of gases at step 306. The flow indicator (for example Kappa) of the flow of gases can be determined based at least in part on the heater plate temperature, the heater plate power, and the ambient temperature, as described above, for example, as a ratio of the heater plate power to the difference between the heater plate temperature and the ambient temperature.

The power offset is only applied when both Kappa drops below a reference flow indicator threshold and the HPTemp exceeds a heater plate temperature threshold, resulting in a positive power offset. In this disclosure, a positive power offset is indicative of a power offset that is applied to the heating element (heater wire). In all other cases, the power offset is set to zero. In this disclosure, a negative power offset is indicative of a power offset that is not applied or set to zero such that the power applied to the heating element is not changed (from for example SSHWDuty).

The process can also determine a difference between a reference flow indicator threshold and the calculated flow indicator (Kappa). The process may cause a power offset on the heating element of the inspiratory conduit to become positive if the flow indicator drops below the reference flow indicator threshold (that is, the system is in a low flow state, for example, at a flow rate under 10 L/min, or under about 5 L/min). The power offset can be subtracted from OffsetHWDuty. A positive power offset will correspondingly cause OffsetHWDuty to be reduced. A larger positive power offset value will cause a larger reduction in OffsetHWDuty. As shown in Figure 4A, an initial drop in the flow can be observed at about the 2 hour time point. The drop in the flow indicator Kappa can be responsible for an initial drop of the OffsetHWDuty of the heating element of the inspiratory conduit. As the flow indicator Kappa further decreases over time, the difference between the reference flow indicator threshold and the flow indicator increases, which may further increase the power offset, which may thereby decrease OffsetHWDuty.

**In** addition to the sudden reduction in flow, the heater plate temperature can also influence whether the power offset is positive. When the heater plate temperature is sufficiently low, which is when the heater plate temperature falls below the threshold described above, the power offset would be zero or negative (which the system can treat the power offset as zero), as the enthalpy is expected to remain below the significant level even in a low flow state regardless of the operation state of the humidifier. The process can also determine a difference between a reference heater plate temperature and the measured heater plate temperature. The process may not apply a positive power offset to OffsetHWDuty unless the measured heater plate temperature is greater than the reference heater plate temperature (and/or the difference between the reference flow indicator threshold and the flow indicator is positive). After the flow indicator Kappa substantially settles, a term including the heater plate temperature can scale the output of the power offset.

Accordingly, to account for the influence of the flow and the heater plate temperature on the power limit, the power offset to the heating element of the inspiratory conduit becomes positive or non-zero only when the heater plate temperature exceeds a temperature threshold and the flow indicator is below the reference flow indicator threshold. To achieve this, as shown in Figure 3A, the hardware controller can calculate a power offset based on the calculated flow indicator and the heater plate temperature at step 308. The power offset can be calculated by multiplying the flow indicator with the heater plate temperature. The power offset may be scaled, optionally by a scaling factor. This can produce a power offset that may be positively (for example, linearly or non-linearly) related to the heater plate temperature when the heater plate temperature is above the predetermined threshold as disclosed herein (see for example the decay of the OffsetHWDuty when the HPTemp initially drops after about the 2 hour time point in Figure 4A).

With continued reference to Figure 3A, at block 310, the hardware controller can clamp the calculated power offset. The power offset can be clamped to 0 (that is, not offsetting the steady state power to the heating element of the inspiratory conduit (SSHWDuty in Figure 4A)) if the heater plate temperature is lower than the heater plate temperature threshold, and/or if the calculated flow indicator is at or exceeds the reference flow indicator threshold, in other words, if the power offset value is calculated to be below 0.

Prior to the time point of about 2 hours, in Section A of Figure 4A, the flow indicator Kappa is greater than the reference flow indicator threshold (the reference flow indicator threshold is not shown in Figure 4A), even though the heater plate temperature HPTemp exceeds the heater plate temperature threshold. Therefore, the power offset is 0. That is, the power output to the heating element of the inspiratory conduit OffsetHWDuty is the same as the SSHWDuty and is below the MaxHWDuty. In this scenario, there is a relatively high flow in the system, with the energization of the heater plate being relatively high compared to the HPTemp.

In Section B, a flow drop occurs, causing the flow indicator Kappa to drop below the reference flow indicator threshold. HPTemp also remains greater than the heater plate temperature threshold. When HPPower drops and HPTemp remains high during the flow drop event, this is an indication of a potential over-enthalpy event. When there is an indication of a potential over-enthalpy event, power to the heating element (heater wire) should be decreased. When the flow indicator Kappa drops below the reference flow indicator threshold and HPTemp remains greater than the heater plate temperature threshold, a positive power offset results, which is subtracted from the SSHWDuty to give OffsetHWDuty.

In Section C, the flow indicator (Kappa) remains below the flow indicator threshold and the HPTemp remains greater than the heater plate temperature threshold, resulting in a positive power offset. A power offset is determined and/or applied by a controller implementing the process in Figure 3A. In Section C, the HPTemp decreases toward the heater plate temperature threshold. This behavior can be used to scale the power offset by decreasing the power offset. A decrease in HPTemp can result in decreased enthalpy at the user end. Therefore, a lower power offset is needed, which can help in reducing unnecessary condensation. Therefore a decrease in HPTemp allows OffsetHWDuty to increase, approximately following the trend of MaxHWDuty.

Finally, in Section D, the system has regained steady-state operation. Therefore, the power offset is set to zero as the HPTemp is below the heater plate temperature threshold despite the flow indicator Kappa still remaining below the reference flow indicator threshold. That is, OffsetHWDuty equals SSHWDuty.

If the power offset is greater than 0, the hardware controller can limit the power to the heating element (for example, the heating element duty cycle) of the inspiratory conduit based on the calculated power offset at step 312. The power offset can be of a magnitude needed to maintain a user end enthalpy limit or temperature (which can be dew point) limit. The limit can be an enthalpy limit of from 122 to 216 kJ/m$^3$, or from 150 to 216 kJ/m$^3$, or from 185 to 210 kJ/m$^3$, or from 195 to 205 kJ/m$^3$. This user end limit can also be a temperature limit (which can be the user end dew point limit) of from 32°C to 50°C, or from 35°C to 45°C, or from 38°C to 42°C.

The power outputted by the hardware controller (labeled as OffsetHWDuty in Figure 4A) can be calculated by subtracting the power offset from the SSHWDuty.

With the power offset being applied to the steady state heater wire power, the temperature of the gases within the inspiratory conduit can be maintained at or below the target enthalpy limit or temperature limit described herein.

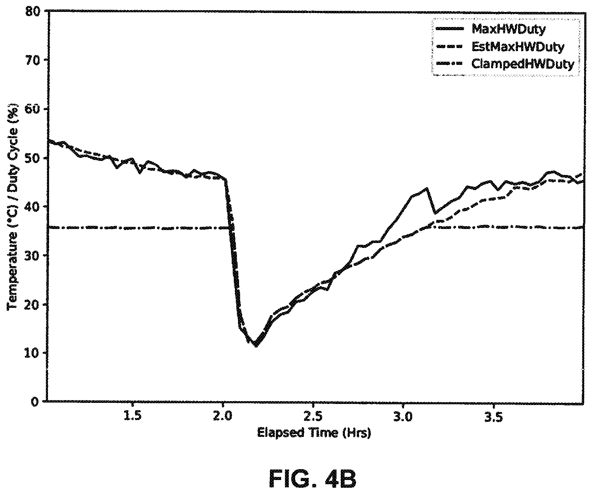

The algorithm can also optionally reduce the power outputted to the heating element of the inspiratory conduit by a greater amount than what is shown in Sections B and C of Figure 4B, or completely turn off the power to the heating element of the inspiratory conduit and/or the heating element of the heater plate.

Method 2 aims to reproduce the MaxHWDuty using parameters available to the humidifier.

Figure 4B shows the MaxHWDuty, an estimated power limit (EstMaxHWDuty), and a power to the heating element of the inspiratory conduit (for example ClampedHWDuty). As described in more detail above, MaxHWDuty is the empirically derived maximum power allowed to meet the safe enthalpy target at the patient end of the inspiratory conduit. EstMaxHWDuty is the power limit calculated via method 2 below. ClampedHWDuty is the power applied to the heater plate.

In normal operation the ClampedHWDuty may be lower than the EstMaxHWDuty as the controller of the heating element of the inspiratory conduit is not attempting to provide a power to the heating element of the inspiratory conduit that exceeds EstMaxHWDuty. In Method 2, the ClampedHWDuty can be set to be the MaxHWDuty (for example during the flow drop, or when the controller of the heating element of the inspiratory conduit attempts to provide a power to the heating element of the inspiratory conduit that exceeds EstMaxHWDuty). Alternatively, the ClampedHWDuty can be set to be lower than the MaxHWDuty (for example during the flow drop, or when the controller of the heating element of the inspiratory conduit attempts to provide a power to the heating element of the inspiratory conduit that exceeds Est-MaxHWDuty). The MaxHWDuty is approximated as the EstMaxHWDuty using parameters available to the humidifier system. The EstMaxHWDuty is calculated by estimating a total humidity generated at the chamber outlet (mg/L). Humidity generation can further be calculated as a function of the flow rate and the rate of evaporation, for example, using the formula: humidity generated (mg/L) = (rate of evaporation (mg/min)) / (flow rate (L/min)).

Here, the flow rate and the rate of evaporation can be further estimated. The flow rate indicator, Kappa, can be estimated as described above in Method 1. Kappa can be a parameter indicative of a flow rate of the flow of gases. The parameter indicative of a flow rate of the flow of gases can include a thermal conductivity value, or a value indicative of a thermal conductivity, between the heater plate and the flow of gases. The value indicative of a thermal conductivity value can be based on the HPPower, HPTemp, and the ambient temperature reading. An estimated rate of evaporation can be calculated as a function of HPTemp ($E_{est}(T_{HP})$). Therefore, a value indicative of humidity can be calculated. For example, a value indicative of humidity, estimated humidity, $H_{est}$, can be calculated using the formula: $H_{est} = (E_{est}(T_{HP}))$ / Kappa. Figure 4C shows a plot of $H_{est}$ that is produced at the chamber outlet calculated using $E_{est}(T_{HP})$/Kappa. This plot can be utilized in calculating the power limit MaxHWDuty.

To calculate the EstMaxHWDuty, the $H_{est}$ can be scaled and offset by factors to match the target power, that is, the MaxHWDuty. ·ambient temperature where $\alpha_1$, $\alpha_2$, and $\alpha_3$ are factors. Alternatively, a variable y can be obtained using the formula: $y = \alpha_1 \cdot H_{est} + \alpha_2$, where $\alpha_1$ and $\alpha_2$ are factors. This variable y is then added to the SSHWDuty to produce the EstMaxHWDuty, which becomes EstMaxHWDuty = $\alpha_1 \cdot H_{est} + \alpha_2$ +SSHWDuty. Factor $\alpha_1$ is negative such that, for a given SSHWDuty, an increase in the $H_{est}$ will decrease the EstMaxHWDuty. This is because $H_{est}$ is used as a proxy for the chamber outlet enthalpy. Any excessive enthalpy needs to be dissipated by the inspiratory conduit via a reduced power to the heating element of the inspiratory conduit. Factor $\alpha_2$ provides an offset to set an allowable humidity level before over-enthalpy is detected. Factor $\alpha_3$ can be negative such that the higher the ambient temperature, the lower the power limit. Method 2's EstMaxHWDuty is valid during steady state operation as well as over-enthalpy events (for example, large flow drops).

Figure 3B illustrates a process for implementing Method 2. As shown, the hardware controller of the system can receive a heater plate temperature value at step 320, an ambient temperature value at step 322, and a heater plate power value at step 324. The hardware controller can calculate a flow indicator of the flow of gases as described above at step 326. At step 328, the hardware controller can calculate an estimated rate of evaporation based on HP temperature. At step 330, the hardware controller can calculate a limit based on dividing $E_{est}$ or the HPTemp by the flow indicator. At step 332, the hardware controller can apply the limit to, by adding to, the SSHWDuty to determine an EstMaxHWDuty, which is an estimate of MaxHWDuty.

In some configurations, the over enthalpy protection can be implemented as a hardware component. In some configurations, the over enthalpy protection can be implemented in software on a controller.

In some configurations, the hardware component for over enthalpy protection can apply a power limit by disabling a component or the entire system if the power limit is exceeded (for example, disabling a heater plate and/or a heating element if the power limit for the heater plate and/or heating element is exceeded). In some configurations, software on a controller can apply a power limit by limiting the power to a heating element of the conduit to, or below, a power limit.

The hardware component for over enthalpy protection may provide a backup for a software over enthalpy protection (for example, where software fails to control the heating element power) having both a hardware component for over enthalpy protection and a software over enthalpy protection in tandem may be useful. As described above the hardware component for over enthalpy protection may protect against a higher level of enthalpy.

As described above, the user may be exposed to a more significant level of enthalpy at the user end of the inspiratory conduit, particularly at lower flow rates, if the temperature of the heater plate is too high, and/or if the power delivered to the heating element of the inspiratory conduit is too high for a given set of conditions.

Enthalpy at the user end of the inspiratory conduit can be kept below a limit using software implementing the process described above. Hardware protection circuits can be used alternative to and/or in addition to the software. The protection circuits can act as redundancy to software, which can provide the primary control against significant enthalpy levels. For example, these protection circuits can act as redundancy to unexpected increases or decreases in the power to the heating element(s) of the heater plate and/or to the inspiratory conduit. These circuit(s) can maintain the user end temperature and/or enthalpy below a certain limit. The limit can be expressed as a specific enthalpy limit (which can be at the user end) of from 122 kJ/m$^3$ to 441 kJ/m$^3$, or from 150 kJ/m$^3$ to 400 kJ/m$^3$, or from 195 kJ/m$^3$ to 350 kJ/m$^3$, or from 195 kJ/m$^3$ to 250 kJ/m$^3$, or from 195 kJ/m$^3$ to 205 kJ/m$^3$. This limit can be expressed as a temperature limit of gases (which can be the user end and the gases can be substantially fully saturated with water vapor) of from 32°C to 70°C, or from 43°C to 63°C, or from 43°C to 50°C, or from 43°C to 45°C. These limits are examples of how an enthalpy limit can be expressed.

The protection circuit can include an ambient temperature input from the ambient temperature sensor described above. The protection circuit can also include a heater plate temperature input from the heater plate temperature sensor described above, or a heater plate power input. The protection circuit can also include an inspiratory conduit heating element power input. The protection circuit can also optionally include a constant offset input. The inputs can be in the form of voltage signals or otherwise. Alternatively, the protection circuit can also be achieved using software processes implemented by the hardware controller that follow the same logic as the protection circuits disclosed herein.

In the respiratory humidifier system disclosed herein, the maximum allowable power to the heating element of the inspiratory conduit can be inversely related to the ambient temperature. When the ambient temperature is higher, a lower power to the heating element of the inspiratory conduit is needed to maintain the same enthalpy and/or temperature of the gases at the user end, for a given set of conditions. Conversely, when the ambient temperature is lower, a higher power to the heating element of the inspiratory conduit is needed to maintain the same enthalpy and/or temperature or the gases at the user end, for a given set of conditions.

The power to the heating element of the inspiratory conduit can be inversely related to the heater plate temperature as the chamber outlet enthalpy is related to the heater plate temperature. When heater plate temperature is higher, a lower power to the heating element of the inspiratory conduit is needed to maintain the same enthalpy and/or temperature of the gases at the user end, for a given set of conditions. Conversely, when the heater plate temperature is lower, a higher power to the heating element of the inspiratory conduit is needed to maintain the same enthalpy and/or temperature or the gases at the user end, for a given set of conditions.

Non-limiting examples of protection circuit configurations are described below. One or more protection circuits can be active at all times. For example, the one or more protection circuits could be implemented as a hardware component.

A first patient end enthalpy protection can be achieved by protection circuits using empirically derived data relating to a given set of conditions. The patient end enthalpy protection is based on heater plate temperature, heater wire power, and ambient temperature. Heater plate temperature, heater wire (or heating element) power, and ambient temperature can be used to determine estimations or approximations of other values such as a value indicative of flow rate of gases or an estimated chamber outlet enthalpy, and the like as described below.

In some configurations, the heater wire power may be based on a heater wire duty cycle, or a heater wire voltage, or a heater wire current and/or heater wire resistance (for example a measured or assumed heater wire resistance known for a particular inspiratory conduit attached.)

The first enthalpy protection may be a steady state protection (as described in more detail below). Steady state may be where the apparatus has been operating for a long enough time such that the HPTemp remains substantially constant, and the system is in thermal equilibrium state. The given set of conditions may relate to a worst case scenario for enthalpy for the system. For example the given set of conditions may be a low flow condition as the volume of gases provided through the system energy from the system, is at its lowest. The protection circuit can take into account the ambient temperature, the power to the heating element of the inspiratory conduit, and the heater plate temperature.

The first enthalpy protection can also be used in non steady state conditions. The first enthalpy protection can be effective where there are small or slow changes in the system conditions. The first enthalpy protection can also be used when heater plate temperature is increasing. The first enthalpy protection can also be used when there is gradual change in heater plate temperature, heater wire power, and ambient temperature. The first enthalpy protection can also be used when there is a gradual change in gas flow rate.

During operation of the system the heater plate power is correlated (for example a fixed correlation) with the difference between heater plate temperature and the water temperature. When the system is at steady state, the heater plate temperature can be used as a proxy for the water temperature because the application of heater plate power is not causing a temperature increase at steady state. This assumption can be used to effectively estimate the chamber outlet enthalpy.

As mentioned above, the power to the heating element of the inspiratory conduit and the ambient temperature can affect how much of the chamber outlet enthalpy reaches the user end, as this determines the amount of energy lost along the inspiratory circuit (for example energy lost via conductive heat loss through the wall of the inspiratory circuit.) The amount of energy which is dissipated along the inspiratory circuit can be determined based on the estimated chamber outlet enthalpy (which can be estimated based on the heater plate temperature as described above), the heater wire power and ambient temperature.

Based on the amount of energy which is dissipated along the inspiratory circuit a maximum allowable power to the heating element of the inspiratory conduit can be determined for a given ambient temperature and estimated chamber outlet enthalpy (which is estimated based on heater plate temperature).

Therefore, for a given heater wire power it can be determined whether the application of that power to the heating element of the inspiratory conduit will cause too much energy to be delivered to the user end, and the protection circuit will be tripped and the power limit applied by disabling the heating element.

Example principles of the protection circuits are described with reference to Figures 5A and 5B. As shown in Figures 5A and 5B, the X-axis represents the heater plate temperature, which effectively represents chamber outlet enthalpy as described above, and the Y-axis represents the power to the heating element of the inspiratory conduit, which effectively represents the amount of the chamber outlet enthalpy transferred through the inspiratory circuit to the user. As explained above, for a given set of conditions (for example, the ambient temperature and/or flow rate), the power to the heating element of the inspiratory conduit can be negatively related to the heater plate temperature to maintain a constant enthalpy at the user end. The negative relationship can be illustrated as a downward sloping line, as shown by line 2 in Figures 5A and 5B.

The downward sloping line (line 2) represents a first boundary of a "safe operating area" (shown by the shaded area) to limit the user end enthalpy to be below an enthalpy limit. As the chamber outlet enthalpy increases (moving to the right of the X-axis), the maximum energy supplied by the inspiratory conduit decreases in order to not exceed the user end enthalpy limit. In Figures 5A and 5B, line 1, which represents a fixed heater plate temperature limit (for example, the heater plate temperature can be limited to about 80°C or otherwise), can provide a second boundary of the "safe operating area." Effectively, the "safe operating area" is defined by the smallest area within all the boundary lines.

As described above, a higher ambient temperature can result in less energy dissipation occurring via the wall of the inspiratory conduit. Line 2 will shift downwards when the ambient temperature increases. Conversely, line 2 will shift upwards when the ambient temperature decreases. Having line 2 as one of the boundary lines can provide an additional benefit in minimizing the risk of over-enthalpy to the user.

As shown in Figure 5B, the "safe operating area" can be further defined by adding additional boundary lines. For example, a horizontal line (line 3) can be added to reduce the maximum allowable power to the heating element of the inspiratory conduit at lower heater plate temperatures. This horizontal line 3 can be a function of the ambient temperature.

The "safe operating area" in Figure 5B can be defined by all three lines 1, 2, and 3, although not all three lines may define the "safe operating area" in all conditions. For example, as the ambient temperature increases, line 2 will shift downwards until lines 1 and 2 no longer intersect above the X-axis (that is, at high ambient temperatures, line 2 can reach zero heater wire power at a lower heater plate temperature than the heater plate temperature corresponding to line 1), such that the "safe operating area" is defined by lines 2 and 3 in this scenario.

Figure 6 illustrates a first enthalpy protection example circuit implementing the principles illustrated in Figures 5A and 5B. As shown, a first conditioning circuit 601 can be connected to the ambient temperature sensor 602. A second conditioning circuit 603 can be connected to the inspiratory conduit heating element ("Heater Wire") power (such as duty cycle) input 604. A third conditioning circuit 605 can be connected to the heater plate temperature sensor 606. A fourth conditioning circuit 607 can be connected to a voltage reference input 608, which acts as an offset term. The conditioning circuits can convert temperature readings to voltage signals for being inputted to the comparator 600. As the temperature sensors may be PTC or NTC sensors, the conditioning circuit may have a negative or positive coefficient accordingly. The sensors and conditioning circuits in Figure 6 are independent of each other. The net polarity of a conditioned signal is the combination of the sensor coefficient and the conditioning circuit coefficient. In a non-limiting example configuration, the circuit can assume the conditioned ambient temperature signal to be negatively related to the ambient temperature; the conditioned inspiratory conduit heating element power signal to be positively related to the power to the heating element of the inspiratory conduit; and the conditioned heater plate temperature signal to be positively related to the heater plate temperature. The circuit compares the ambient temperature signal with a combination of the heater plate temperature signal, the HW power signal, and the offset voltage signal by using a comparator 600, for example, by comparing two voltage inputs to the comparator 600, which are an input from the first conditioning circuit and a combination of inputs from the second, third, and fourth conditioning circuits, 603, 605, 607.

In some configurations, the comparator is a hardware component. It is understood that any hardware component that can compare signals can be used. In some configurations, the comparator is implemented in software of a controller comparing input signals.

The output of the comparator, HW_OVERDUTY 610, is an indicator that the system is trying to deliver more than the maximum allowable power to the inspiratory conduit for the given conditions. This signal can be used to control the state of the switch to the heater element of the inspiratory conduit and/or the switch to the heater plate heating element. Under normal operating conditions, the power to the heating element of the inspiratory conduit is less than the maximum allowable power limit, so HW_OVERDUTY is always low under normal operating conditions.

Another form of patient end enthalpy protection (as a second patient end enthalpy protection) is described below. As explained above, one of the factors relating to enthalpy in the humidifier system is the temperature of the water in the chamber (for example, the humidifier chamber 103 of Figure 1A). However, many humidifier systems do not detect the water temperature directly. Rather, a heater plate temperature and a heater plate power may be used to indirectly infer or estimate the water temperature. As the water temperature determines the evaporation rate and hence the energy transfer rate, and enthalpy is defined as energy per volume, enthalpy can be limited by limiting the water temperature.

As described above the difference between the heater plate temperature and water temperature is proportional (for example positively related) to the heater plate power. The relationship between the heater plate temperature, water temperature and heater plate power can be approximated as:

$$T_{HP} - T_{H_2O} \propto P_{HP}$$

The difference between the heater plate temperature and the water temperature is a result of imperfect heat conduction between the heater plate and the water in the chamber. As described above, during operation of the system the heater plate power is correlated to the difference between the heater plate temperature and water temperature. This correlation can be empirically determined for a set of operating parameters that reaches a specific level of enthalpy (for example, when the humidifier system is operating in a steady state). However, this assumption becomes invalid when the system is not in steady state, since the heater plate power is higher or lower than the steady state power to cause temperature changes with time. Therefore, the water temperature cannot be determined by the heater plate temperature alone when the system is not in a steady state. An example situation of when the system is not in a steady state is when there is a large change in flow rate of the gases.

For a given heater plate temperature, the water temperature will be lower than the assumed water temperature if the heater plate power is higher than the assumed power level. Therefore, the chamber outlet enthalpy will also be lower than assumed, which means that the steady state protection described above will be sufficient in protecting against the lower enthalpy level in this scenario (for example when the HPTemp is increasing and the water temperature is additionally based on how much power is applied to the heater plate).

However, for a given heater plate temperature, the water temperature will be higher than the assumed water temperature if the heater plate power is lower than the assumed power level. Therefore, the chamber outlet enthalpy will also be higher than assumed, which means that further protection may be desirable.

Put another way, for a given water temperature, the heater plate temperature may be lower than the assumed heater plate temperature, which in turn leads the steady state protection to accept a higher power to the heating element of the inspiratory conduit. This can be undesirable as the power to the heating element of the inspiratory conduit affects how much chamber outlet enthalpy is transferred to the user end. Therefore, an ideal maximum power limit to the heating element of the inspiratory conduit should be related to the water temperature and remain constant if the water temperature remains constant. As shown in Figure 7, when the heater plate power (HPDC) drops significantly, the heater plate temperature (Thp) has also dropped significantly. As the water temperature (Twater) has not dropped to the same extent as the heater plate temperature (Thp), the maximum power to the heating element of the inspiratory conduit ideally should also stay relatively constant in this period. However, the drop in the heater plate temperature has led to an increase in the maximum allowable power to the heating element of the inspiratory conduit (HWDCLimit1) allowed by the steady state protection circuit (as described in more detail above). Therefore, if only the above steady state protection circuit is used the maximum allowable power to the heating element of the inspiratory conduit may therefore exceed an ideal maximum power limit to the heating element of the inspiratory conduit, which corresponds to an empirically derived maximum allowable user end enthalpy limit (IdealLimit), meaning that the steady state protection in isolation may not sufficiently protecting against more significant user end enthalpy in this scenario.

To further protect against the more significant user end enthalpy and for example when the system is not in steady state, the heater plate power input in the steady state protection circuit can be used to estimate the water temperature according to the equation described above, rather than relying on the fixed correlation between the heater plate temperature and the water temperature.

The equation can be re-arranged to make the water temperature the subject:

$$T_{H_2O} \approx T_{HP} - W * P_{HP}$$

Where W is a coefficient which corresponds to the thermal resistance between the heater plate and the humidifier chamber.

The equation allows the chamber outlet enthalpy to be predicted more accurately and provides sufficient protection against the more significant user end enthalpy (for example at lower heater plate powers). Sufficient protection means that for any given scenario, the power to the heating element of the inspiratory conduit does not exceed the ideal power limit to the heating element of the inspiratory conduit that corresponds to a maximum user end enthalpy limit. As shown in Figure 8, an example protection circuit that additionally incorporates the heater plate power input results in a maximum allowable power to the heating element of the inspiratory conduit (HWDCLimit2) that never exceeds the ideal power limit to the heating element of the inspiratory conduit that corresponds to a maximum user end enthalpy limit (IdealLimit).

A protection circuit for a second patient end enthalpy protection, which incorporates the heater plate power input (as well as a heater plate temperature input) is shown in Figure 9. As shown, a first conditioning circuit 1001 can be connected to the ambient temperature sensor 1002. A second conditioning circuit 1003 can be connected to the heater plate temperature sensor 1004. A third conditioning circuit 1005 can be connected to the heater plate power input 1006. A fourth conditioning circuit 1007 can be connected to the inspiratory conduit heating element ("Heater Wire") power (such as duty cycle) input 1008. A fifth conditioning circuit 1009 can be connected to a voltage reference 1010, which acts as an offset circuit. The conditioning circuits convert temperatures or powers to voltage signals for being inputted to the comparator 1000. The temperature sensors may be PTC or NTC thermistors, and the conditioning circuit may have a negative or positive coefficient. The sensors and conditioning circuits in Figure 9 are independent of each other. The net polarity of a conditioned signal is the combination of the sensor coefficient and the conditioning circuit coefficient.

The third conditioning circuit 1005 is connected to the negative input terminal of the comparator 1000. The protection circuit compares the combination of the conditioned ambient temperature signal and the conditioned heater plate temperature signal with a combination of the conditioned HW power signal, the conditioned heater plate power signal, and the offset voltage reading by using a comparator 1000, for example, by comparing two voltage inputs to the comparator 1000, which are inputs from the third, fourth, and fifth conditioning circuits 1005, 1007, 1009 and inputs from the first and second conditioning circuits, 1001, 1003.

Input from the third conditioning circuit 1005 in the second example circuit limits the power limit on the heating element of the inspiratory conduit when the heater plate temperature decreases. The other inputs, such as the ambient temperature input and the offset input, can act as a base value for the power limit on the heating element of the inspiratory conduit.

The output of the comparator, OVER_ENTHALPY 1020 can be configured to directly or indirectly enable or disable power to the heating element of the inspiratory conduit and/or heater plate. For example, the output of the comparator, OVER_ENTHALPY 1020, is used to indirectly control the state of the switch to the heating element of the inspiratory conduit and/or the switch to the heater plate heating element. In this example, under steady state conditions, OVER_ENTHALPY 1020 is binary 0 (or LOW) and does not disable power to the heating element of the inspiratory conduit and/or heater plate, whereas when the circuit is triggered, OVER_ENTHALPY 1020 becomes binary 1 (or HIGH), which can be configured to disable power to the inspiratory conduit and/or heater plate. When the heater plate power decreases, the voltage reading on the negative terminal decreases. This lowers the threshold at which the voltage reading at the positive terminal can exceed the negative terminal. In other words, when the heater plate power decreases, the power limit to the heating element of the inspiratory conduit also decreases. In some configurations, the output of the comparator may be a control output (rather than a binary output as described above) for controlling the power to the heating element of the inspiratory conduit and/or the heater plate.

The heater plate power input in the second enthalpy protection example circuit limits the power limit on the heating element of the inspiratory conduit when the heater plate power decreases. When the second enthalpy protection example circuit acts on its own, the power limit to the heating element of the inspiratory conduit may increase when the heater plate power increases.

**In** contrast, the first enthalpy protection circuit described above limits the power limit on the heating element of the inspiratory conduit based at least in part on the heater plate temperature. Accordingly, the second enthalpy protection example circuit can be implemented in combination with the first enthalpy protection circuit.

**In** some configurations, the first patient end enthalpy protection can be implemented in hardware without any software enthalpy protection (for example, the first patient end enthalpy protection circuit). In some configurations, the second patient end enthalpy protection can be implemented in hardware without any software enthalpy protection (for example, the second patient end enthalpy protection circuit). In some configurations, the first patient end enthalpy protection and the second patient end enthalpy protection can be implemented in hardware without any software enthalpy protection

In some configurations, respiratory humidifier system can have a first power limit and a second power limit on the conduit heating element and/or the one or more heating elements of the heater plate based at least on one or more parameters of the heater plate. In some configurations, the first enthalpy protection (for example by the first enthalpy protection circuit) can be used to determine and/or apply a first power limit. In some configurations, the second protection (for example by the second enthalpy protection example circuit) can be used to determine/or apply a second power limit. The first enthalpy protection example circuit (the first power limit) can be used to control to, or below, the first power limit. The second enthalpy protection example circuit (the second power limit) can disable power to the conduit heating element. Alternatively or in addition, the second enthalpy protection can disable power to one or more heater plate heating elements. In some configurations, the threshold to enact the first power limit is lower than the threshold to enact the second power limit. However, as described above it will be appreciated that the example circuits may be implemented in software. In some configurations, the lower threshold of the first power limit (for example, the first enthalpy protection example circuit) can attempt to limit power to the conduit heating element to, or below, a power limit. In some configurations, if limiting power to the conduit heating element to, or below, a power limit is insufficient to lower patient end enthalpy to a safe level, then the second enthalpy protection example circuit will disable power to the conduit heating element and/or one or more heater plate heating elements. The second power limit of the second enthalpy protection can have a higher threshold than the first power limit of the first enthalpy protection. In some configurations, the first power limit corresponds with a lower patient end enthalpy limit than the second power limit.

The above-described example circuits can act independently to one another as they are separate hardware circuits. If at least one of the hardware circuits are triggered when implemented in combination, power to the heating element of the inspiratory conduit and/or heater plate heating element can be disabled (for example power to the heating element of the inspiratory conduit and/or the heater plate heating element may be disabled even when either one of the hardware circuits has not been triggered.). If neither hardware circuits are triggered, power to the heating element of the inspiratory conduit and/or heater plate heating element will not be disabled. Even though the power limit on the heating element of the inspiratory conduit may shift up when the heater plate power increases due to the abrupt change enthalpy protection example circuit, the first enthalpy protection circuit can protect against such an increase in the power limit on the heating element of the inspiratory conduit.

Terminology

Examples of respiratory humidification systems and associated components and methods have been described with reference to the figures. The figures show various systems and modules and connections between them. The various modules and systems can be combined in various configurations and connections between the various modules and systems can represent physical or logical links. The representations in the figures have been presented to clearly illustrate the principles and details regarding divisions of modules or systems have been provided for ease of description rather than attempting to delineate separate physical configurations. The examples and figures are intended to illustrate and not to limit the scope of the inventions described herein. For example, the principles herein may be applied to a respiratory humidifier as well as other types of humidification systems, including surgical humidifiers.

As used herein, the term "processor" refers broadly to any suitable device, logical block, module, circuit, or combination of elements for executing instructions. For example, the controller 8 can include any conventional general purpose single- or multi-chip microprocessor such as a Pentium® processor, a MIPS® processor, a Power PC® processor, AMD® processor, ARM® processor, or an ALPHA® processor. In addition, the controller 122 can include any conventional special purpose microprocessor such as a digital signal processor or a microcontroller. The various illustrative logical blocks, modules, and circuits described in connection with the configurations disclosed herein can be implemented or performed with a general purpose processor, a digital signal processor (DSP), an application specific integrated circuit (ASIC), a field programmable gate array (FPGA), or other programmable logic device, discrete gate or transistor logic, discrete hardware components, or any combination thereof designed to perform the functions described herein, or can be a pure software executed by the main processor. For example, logic module can be a software-implemented function block which does not utilize any additional and/or specialized hardware elements. Controller can be implemented as a combination of computing devices, for example, a combination of a DSP and a microprocessor, a combination of a microcontroller and a microprocessor, a plurality of microprocessors, one or more microprocessors in conjunction with a DSP core, or any other such configuration.

Data storage can refer to electronic circuitry that allows data to be stored and retrieved by a processor. Data storage can refer to external devices or systems, for example, disk drives or solid state drives. Data storage can also refer to solid state semiconductor storage (chips), for example, Random Access Memory (RAM) or various forms of Read Only Memory (ROM), which are directly connected to the communication bus or the controller 8. Other types of data storage include bubble memory and core memory. Data storage can be physical hardware configured to store data in a non-transitory medium.

The scope of the claims or configurations appended hereto is not limited by any of the particular configurations described herein. For example, in any method or process disclosed herein, the acts or operations of the method or process can be performed in any suitable sequence and are not necessarily limited to any particular disclosed sequence. Various operations can be described as multiple discrete operations in turn, in a manner that can be helpful in understanding certain configurations; however, the order of description should not be construed to imply that these operations are order dependent. Additionally, the structures described herein can be embodied as integrated components or as separate components. For purposes of comparing various configurations, certain aspects and advantages of these configurations are described. Not necessarily all such aspects or advantages are achieved by any particular configuration. Thus, for example, various configurations can be carried out in a manner that achieves or optimizes one advantage or group of advantages as taught herein without necessarily achieving other aspects or advantages as can also be taught or suggested herein.

Conditional language used herein, such as, among others, "can," "could," "might," "may," "e.g.," and the like, unless specifically stated otherwise, or otherwise understood within the context as used, is generally intended to convey that certain configurations include, while other configurations do not include, certain features, elements and/or states. Thus, such conditional language is not generally intended to imply that features, elements and/or states are in any way required for one or more configurations. As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Also, the term "or" is used in its inclusive sense (and not in its exclusive sense) so that when used, for example, to connect a list of elements, the term "or" means one, some, or all of the elements in the list. Conjunctive language such as the phrase "at least one of X, Y and Z," unless specifically stated otherwise, is otherwise understood with the context as used in general to convey that an item, term, etc. may be either X, Y or Z. Thus, such conjunctive language is not generally intended to imply that certain configurations require at least one of X, at least one of Y and at least one of Z each to be present. As used herein, the words "about" or "approximately" can mean a value is within ±10%, within ±5%, or within ±1% of the stated value.

Methods and processes described herein may be embodied in, and partially or fully automated via, software code modules executed by one or more general and/or special purpose computers. The word "module" refers to logic embodied in hardware and/or firmware, or to a collection of software instructions, possibly having entry and exit points, written in a programming language, such as, for example, C or C++. A software module may be compiled and linked into an executable program, installed in a dynamically linked library, or may be written in an interpreted programming language such as, for example, BASIC, Perl, or Python. It will be appreciated that software modules may be callable from other modules or from themselves, and/or may be invoked in response to detected events or interrupts. Software instructions may be embedded in firmware, such as an erasable programmable read-only memory (EPROM). It will be further appreciated that hardware modules may comprise connected logic units, such as gates and flip-flops, and/or may comprised programmable units, such as programmable gate arrays, application specific integrated circuits, and/or processors. The modules described herein can be implemented as software modules, but also may be represented in hardware and/or firmware. Moreover, although in some configurations a module may be separately compiled, in other configurations a module may represent a subset of instructions of a separately compiled program, and may not have an interface available to other logical program units.

In certain configurations, code modules may be implemented and/or stored in any type of computer-readable medium or other computer storage device. In some systems, data (and/or metadata) input to the system, data generated by the system, and/or data used by the system can be stored in any type of computer data repository, such as a relational database and/or flat file system. Any of the systems, methods, and processes described herein may include an interface configured to permit interaction with users, operators, other systems, components, programs, and so forth.

It should be emphasized that many variations and modifications may be made to the configurations described herein, the elements of which are to be understood as being among other acceptable examples. All such modifications and variations are intended to be included herein within the scope of this disclosure. Further, nothing in the foregoing disclosure is intended to imply that any particular component, characteristic or process step is necessary or essential. The invention is defined by the claims which follow.

**Claims**

1. A respiratory or surgical humidifier system (100; 200; 10; 1) configured to deliver a flow of gases to a user, the system comprising:

   a base unit comprising:

   a heater plate (120; 212) including one or more heater plate heating elements;
   a hardware controller (208a, 208b: 208; 13) configured to energize the one or more heating elements of the heater plate; and
   a protection circuit,

   the base unit configured to receive a humidifier chamber including a thermally conductive base such that the thermally conductive base contacts the heater plate (120; 212), the humidifier chamber configured to hold a volume of water, wherein the hardware controller (208a, 208b: 208; 13) is configured to be in electronic communication with a conduit heating element in an inspiratory conduit configured to transport the gases from the humidifier chamber to a patient interface,
   the system (100; 200; 10; 1) configured to apply a first power limit and a second power limit on the conduit heating element based at least in part on one or more parameters of the heater plate (120; 212);
   wherein the first power limit is applied by the hardware controller (208a, 208b: 208; 13) and the second power limit is applied by the protection circuit.

2. The system of claim **1,** wherein the first power limit is controlling to, or below, a power limit.

3. The system of claim 1 or claim 2, wherein the second power limit is disabling power to the conduit heating element.

4. The system of any one of claims 1-3, wherein a threshold to enact the first power limit is lower than a threshold to enact the second power limit.

5. The system of any one of claims 1-4, wherein the first power limit corresponds with a lower patient end enthalpy limit than the second power limit; and/or
   wherein the first power limit is configured to keep a user end enthalpy of the gases under a first enthalpy limit on a specific enthalpy for dry air and the second power limit is configured to keep a user end enthalpy of the gases under a second enthalpy limit on a specific enthalpy for dry air.

6. The system of any one of claims 1-5, wherein applying a first power limit on the conduit heating element comprises controlling power in the conduit heating element to, or below the first power limit.

7. The system of any one of claims 1-6, wherein applying a second power limit on the conduit heating element comprises disabling a component of the system or the entire system if the second power limit on the conduit heating element is exceeded.

8. The system of claim 7, wherein disabling a component of the system comprises disabling the conduit heating element and/or disabling the heater plate.

9. The system of any one of claims 1-8, comprising a heater plate temperature sensor configured to measure a heater plate temperature and the one or more parameters of the heater plate, which the system is configured to apply the first power limit and/or the second power limit based at least in part on, includes at least the heater plate temperature.

10. The system of any one of claims 1-9, comprising an ambient temperature sensor configured to measure an ambient temperature and the system is configured to apply the first power limit and/or the second power limit on the conduit heating element based in part on the ambient temperature.

11. The system of any one of claims 1-10, wherein the one or more parameters of the heater plate, which the system is configured to determine and/or apply the first power limit and/or the second power limit based at least in part on, includes at least a heater plate power.

12. The system of claim 11 wherein the hardware controller is configured to apply the first power limit based at least in part on a heater plate power and the protection circuit is configured to apply the second power limit based at least in part on the heater plate power.

13. The system of claim 12 further comprising a second protection circuit comprising a comparator.

14. The system of claim 13 wherein the second protection circuit is configured to apply a power limit based at least in part on heater plate temperature.

15. The system of any one of claims 1-14, wherein the system is configured to apply the first power limit and/or the second power limit:

    a) without requiring input from sensors in a gases flow pathway; and/or
    b) without requiring input from sensors located at or near a chamber outlet and/or a patient end.


**Patentansprüche**

1. Befeuchtungssystem für Atemwege oder Operationssäle (100; 200; 10; 1), das so konfiguriert ist, dass es einem Benutzer einen Gasstrom zuführt, wobei das System umfasst:

    eine Basiseinheit, umfassend:

        eine Heizplatte (120; 212), die ein oder mehrere Heizplatten-Heizelemente beinhaltet;
        eine Hardware-Steuereinheit (208a, 208b: 208; 13), die so konfiguriert ist, dass sie ein oder mehrere Heizelemente der Heizplatte mit Energie versorgt; und
        eine Schutzschaltung,

    wobei die Basiseinheit so konfiguriert ist, dass sie eine Befeuchterkammer, die eine wärmeleitende Basis beinhaltet, so aufnimmt, dass die wärmeleitende Basis die Heizplatte (120; 212) berührt, wobei die Befeuchterkammer so konfiguriert ist, dass sie ein Wasservolumen hält, wobei die Hardware-Steuereinheit (208a, 208b: 208; 13) so konfiguriert ist, dass sie in elektronischer Kommunikation mit einem Leitungsheizelement in einer Inspirationsleitung steht, die so konfiguriert ist, dass sie die Gase von der Befeuchterkammer zu einer Patientenschnittstelle transportiert,
    wobei das System (100; 200; 10; 1) so konfiguriert ist, dass es eine erste Leistungsgrenze und eine zweite Leistungsgrenze auf das Leitungsheizelement auf der Grundlage zumindest teilweise eines oder mehrerer Parameter der Heizplatte (120; 212) anwendet;
    wobei die erste Leistungsgrenze von der Hardware-Steuereinheit (208a, 208b: 208; 13) und die zweite Leistungsgrenze von der Schutzschaltung angewendet wird.

2. System nach Anspruch 1, wobei die erste Leistungsgrenze Steuern auf eine Leistungsgrenze oder darunter ist.

3. System nach Anspruch 1 oder Anspruch 2, wobei die zweite Leistungsgrenze Abschalten einer Leistung zum Leitungsheizelement ist.

4. System nach einem der Ansprüche 1-3, wobei ein Schwellenwert zum Durchsetzen der ersten Leistungsgrenze niedriger ist als der Schwellenwert zum Durchsetzen der zweiten Leistungsgrenze.

5. System nach einem der Ansprüche 1-4, wobei die erste Leistungsgrenze einer niedrigeren Patientenend-Enthalpiegrenze entspricht als die zweite Leistungsgrenze; und/oder
wobei die erste Leistungsgrenze so konfiguriert ist, dass eine Benutzer-Endenthalpie der Gase unter einer ersten Enthalpiegrenze für eine bestimmte Enthalpie für trockene Luft gehalten wird, und die zweite Leistungsgrenze so konfiguriert ist, dass eine Benutzer-Endenthalpie der Gase unter einer zweiten Enthalpiegrenze für eine bestimmte Enthalpie für trockene Luft gehalten wird.

6. System nach einem der Ansprüche 1-5, wobei Anwenden einer ersten Leistungsgrenze auf das Leitungsheizelement Steuern einer Leistung im Leitungsheizelement auf oder unter die erste Leistungsgrenze umfasst.

7. System nach einem der Ansprüche 1-6, wobei Anwenden einer zweiten Leistungsgrenze auf das Leitungsheizelement Deaktivieren einer Komponente des Systems oder des gesamten Systems umfasst, wenn die zweite Leistungsgrenze für das Leitungsheizelement überschritten wird.

8. System nach Anspruch 7, wobei Deaktivieren einer Komponente des Systems Deaktivieren des Leitungsheizelements und/oder Deaktivieren der Heizplatte umfasst.

9. System nach einem der Ansprüche 1-8, das einen Heizplattentemperatursensor umfasst, der zum Messen einer Heizplattentemperatur konfiguriert ist, und der eine oder die mehreren Parameter der Heizplatte zumindest die Heizplattentemperatur beinhalten, wobei das System konfiguriert ist, zumindest teilweise auf deren Grundlage die erste Leistungsgrenze und/oder die zweite Leistungsgrenze anzuwenden.

10. System nach einem der Ansprüche 1-9, das einen Umgebungstemperatursensor umfasst, der zum Messen einer Umgebungstemperatur konfiguriert ist, und das System so konfiguriert ist, dass es die erste Leistungsgrenze und/oder die zweite Leistungsgrenze auf das Leitungsheizelement auf der Grundlage zumindest teilweise der Umgebungstemperatur anwendet.

11. System nach einem der Ansprüche 1-10, wobei der eine oder die mehreren Parameter der Heizplatte zumindest eine Heizplattenleistung beinhalten, wobei das System konfiguriert ist, die erste Leistungsgrenze und/oder die zweite Leistungsgrenze auf zumindest teilweise deren Grundlage zu bestimmen und/oder anzuwenden.

12. System nach Anspruch 11, wobei die Hardware-Steuereinheit so konfiguriert ist, dass sie die erste Leistungsgrenze auf der Grundlage zumindest teilweise der Heizplattenleistung anwendet, und die Schutzschaltung so konfiguriert ist, dass sie die zweite Leistungsgrenze auf der Grundlage zumindest teilweise der Heizplattenleistung anwendet.

13. System nach Anspruch 12, das ferner eine zweite Schutzschaltung umfasst, die einen Komparator umfasst.

14. System nach Anspruch 13, wobei die zweite Schutzschaltung so konfiguriert ist, dass sie eine Leistungsgrenze auf der Grundlage zumindest teilweise der Heizplattentemperatur anwendet.

15. System nach einem der Ansprüche 1-14, wobei das System so konfiguriert ist, dass es die erste Leistungsgrenze und/oder die zweite Leistungsgrenze anwendet:

   a) ohne Eingaben von Sensoren in einem Gasstrompfad zu benötigen; und/oder
   b) ohne Eingaben von Sensoren zu benötigen, die sich an oder nahe bei einem Kammerausgang und/oder einem Patientenende befinden.

**Revendications**

1. Système d'humidificateur respiratoire ou chirurgical (100 ; 200 ; 10 ; 1) configuré pour fournir un flux de gaz à un utilisateur, le système comprenant :

   une unité de base comprenant :

une plaque chauffante (120 ; 212) incluant un ou plusieurs éléments chauffants de plaque chauffante ;
un dispositif de commande matériel (208a, 208b : 208 ; 13) configuré pour alimenter les un ou plusieurs éléments chauffants de la plaque chauffante ; et
un circuit de protection,

l'unité de base étant configurée pour recevoir une chambre d'humidificateur incluant une base thermoconductrice de sorte que la base thermoconductrice entre en contact avec la plaque chauffante (120 ; 212), la chambre d'humidificateur étant configurée pour contenir un volume d'eau, dans lequel le dispositif de commande matériel (208a, 208b ; 208 ; 13) est configuré pour être en communication électronique avec un élément chauffant de conduit dans un conduit inspiratoire configuré pour transporter les gaz de la chambre d'humidificateur vers une interface patient,
le système (100 ; 200 ; 10 ; 1) étant configuré pour appliquer une première limite de puissance et une seconde limite de puissance sur l'élément chauffant de conduit, sur la base au moins en partie d'un ou de plusieurs paramètres de la plaque chauffante (120 ; 212) ;
dans lequel la première limite de puissance est appliquée par le dispositif de commande matériel (208a, 208b ; 208 ; 13) et la seconde limite de puissance est appliquée par le circuit de protection.

2. Système selon la revendication 1, dans lequel la première limite de puissance est la commande à une limite de puissance inférieure ou égale à une limite de puissance.

3. Système selon la revendication 1 ou la revendication 2, dans lequel la seconde limite de puissance est la désactivation de l'alimentation de l'élément chauffant de conduit.

4. Système selon l'une quelconque des revendications 1-3, dans lequel un seuil pour mettre en œuvre la première limite de puissance est inférieur à un seuil pour mettre en œuvre la seconde limite de puissance.

5. Système selon l'une quelconque des revendications 1-4, dans lequel la première limite de puissance correspond à une limite d'enthalpie finale de patient inférieure à la seconde limite de puissance ; et/ou
dans lequel la première limite de puissance est configurée pour maintenir une enthalpie finale d'utilisateur des gaz sous une première limite d'enthalpie sur une enthalpie spécifique pour l'air sec et la seconde limite de puissance est configurée pour maintenir l'enthalpie finale d'utilisateur des gaz sous une seconde limite d'enthalpie sur une enthalpie spécifique pour l'air sec.

6. Système selon l'une quelconque des revendications 1-5, dans lequel l'application d'une première limite de puissance sur l'élément chauffant de conduit comprend la commande de la puissance dans l'élément chauffant de conduit à une valeur inférieure ou égale à la première limite de puissance.

7. Système selon l'une quelconque des revendications 1-6, dans lequel l'application d'une seconde limite de puissance sur l'élément chauffant de conduit comprend la désactivation d'un composant du système ou du système entier si la seconde limite de puissance sur l'élément chauffant de conduit est dépassée.

8. Système selon la revendication 7, dans lequel la désactivation d'un composant du système comprend la désactivation de l'élément chauffant de conduit et/ou la désactivation de la plaque chauffante.

9. Système selon l'une quelconque des revendications 1-8, comprenant un capteur de température de plaque chauffante configuré pour mesurer une température de plaque chauffante et les un ou plusieurs paramètres de la plaque chauffante, sur la base au moins en partie desquels le système est configuré pour appliquer la première limite de puissance et/ou la seconde limite de puissance, incluent au moins la température de la plaque chauffante.

10. Système selon l'une quelconque des revendications 1-9, comprenant un capteur de température ambiante configuré pour mesurer une température ambiante, et le système est configuré pour appliquer la première limite de puissance et/ou la seconde limite de puissance sur l'élément chauffant de conduit sur la base en partie de la température ambiante.

11. Système selon l'une quelconque des revendications 1-10, dans lequel les un ou plusieurs paramètres de la plaque chauffante, sur la base au moins en partie desquels le système est configuré pour déterminer et/ou appliquer la première limite de puissance et/ou la seconde limite de puissance, incluent au moins la puissance de plaque chauffante.

**12.** Système selon la revendication 11, dans lequel le dispositif de commande matériel est configuré pour appliquer la première limite de puissance sur la base au moins en partie d'une puissance de plaque chauffante, et le circuit de protection est configuré pour appliquer la seconde limite de puissance sur la base au moins en partie de la puissance de plaque chauffante.

**13.** Système selon la revendication 12 comprenant en outre un second circuit de protection comprenant un comparateur.

**14.** Système selon la revendication 13, dans lequel le second circuit de protection est configuré pour appliquer une limite de puissance sur la base au moins en partie de la température de plaque chauffante.

**15.** Système selon l'une quelconque des revendications 1-14, dans lequel le système est configuré pour appliquer la première limite de puissance et/ou la seconde limite de puissance :

a) sans nécessiter d'entrée de capteurs dans une voie de flux de gaz ; et/ou
b) sans nécessiter d'entrée de capteurs situés au niveau ou près d'une sortie de chambre et/ou d'une extrémité de patient.

**FIG. 1A**

**FIG. 1B**

**FIG. 1C**

**FIG. 2A**

**FIG. 2A2**

**FIG. 2B**

**FIG. 2B2**

**FIG. 2C**

**FIG. 2C2**

**FIG. 2D**

FIG. 2E

**FIG. 3**

```
┌─────────────────────────────────┐ ⌐ 300
│      Receive HPTemp Reading      │
└─────────────────────────────────┘
                 │
                 ▼
┌─────────────────────────────────┐ ⌐ 302
│  Receive Ambient Temperature Reading  │
└─────────────────────────────────┘
                 │
                 ▼
┌─────────────────────────────────┐ ⌐ 304
│       Receive HPPower Value      │
└─────────────────────────────────┘
                 │
                 ▼
┌─────────────────────────────────┐ ⌐ 306
│       Calculate Flow Indicator   │
└─────────────────────────────────┘
                 │
                 ▼
┌─────────────────────────────────┐ ⌐ 308
│ Calculate Offset by Multiplying Flow Indicator and │
│               HPTemp             │
└─────────────────────────────────┘
                 │
                 ▼
┌─────────────────────────────────┐ ⌐ 310
│            Clamp Offset          │
└─────────────────────────────────┘
                 │
                 ▼
┌─────────────────────────────────┐ ⌐ 312
│   Apply Clamped Offset to SSHWDuty   │
└─────────────────────────────────┘
```

# FIG. 3A

FIG. 3B

**FIG. 4A**

FIG. 4B

**FIG. 4C**

**FIG. 5A**

**FIG. 5B**

**FIG. 6**

FIG. 7

**FIG. 8**

FIG. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2020009347 A1